# EUROPÄISCHE PATENTSCHRIFT

(11) **EP 3 874 021 B1**
(45) Veröffentlichungstag und Bekanntmachung des Hinweises auf die Patenterteilung: **02.03.2022**
(21) Anmeldenummer: 19802089.3
(22) Anmeldetag: 28.10.2019
(51) Int. Cl.: C12M 3/00, C12M 1/00, C12M 1/12, C12M 1/42

(54) **FLUIDIKVORRICHTUNG, FLUIDIKSYSTEM UND VERFAHREN ZUM ENTWICKELN DREIDIMENSIONALER ZELLULÄRER GEBILDE**
FLUIDIC DEVICE, FLUIDIC SYSTEM, AND METHOD FOR DEVELOPING THREE-DIMENSIONAL CELLULAR CONSTRUCTIONS
DISPOSITIF FLUIDIQUE, SYSTÈME FLUIDIQUE ET PROCÉDÉ DE DÉVELOPPEMENT DE STRUCTURES CELLULAIRES TRIDIMENSIONNELLES

(30) Priorität: 02.11.2018 DE 102018127406
(43) Veröffentlichungstag der Anmeldung: 08.09.2021
(73) Patentinhaber: Technische Universität Darmstadt, 64289 Darmstadt (DE)
(72) Erfinder: NUBER, Ulrike, 64367 Mühltal (DE); KOEPPL, Heinz, 64287 Darmstadt (DE); HARDT, Steffen, 64846 Gross-Zimmern (DE); TÖPFER, Katrin, 64297 Darmstadt (DE)
(74) Vertreter: Fuchs Patentanwälte Partnerschaft mbB
(86) Internationale Anmeldenummer: PCT/EP2019/079418
(87) Internationale Veröffentlichungsnummer: WO 2020/089178

(56) Entgegenhaltungen:
- EP-A1- 3 029 135
- WO-A1-2014/090993
- WO-A1-2017/083705
- US-A1- 2007 042 490
- US-A1- 2008 261 298
- US-A1- 2015 298 123
- US-A1- 2017 240 854
- US-A1- 2018 030 409
- US-B2- 9 034 640

## Beschreibung

Die vorliegende Erfindung betrifft eine Fluidikvorrichtung, ein Fluidiksystem und ein Verfahren zum Entwickeln eines zellulären Ausgangsmaterials zu einem oder mehreren dreidimensionalen zellulären Gebilde/n, unter Beaufschlagung des zellulären Ausgangsmaterials mit wenigstens einem Konzentrationsgradienten.

### Stand der Technik

Derzeit stellt eine mangelnde Verfügbarkeit vitalen Patientengewebes ein Hindernis in der medizinischen Forschung dar. Dies gilt insbesondere für Gewebe des zentralen Nervensystems, wie z.B. Gehirngewebe. Aus diesem Grund werden verschiedene Arten von Stamm- oder Vorläuferzellen (beispielsweise induzierte pluripotente Stammzellen (iPS-Zellen), d.h. aus Gewebe entnommene und zu Stammzellen reprogrammierte Zellen, embryonale Stammzellen (ES-Zellen), Organ-spezifische Stammzellen) zur Züchtung von Zellkulturen als Krankheitsmodell verwendet. Allerdings werden solche Zellkulturen bisher hauptsächlich als 2D-Kulturen gezüchtet. 3D-Kulturen (beispielsweise Organoide), die das Potential besitzen aussagekräftigere Ergebnisse zu liefern, können bisher nicht oder nur in unzureichender Qualität und zu kleinen Dimensionen hergestellt werden. Dies gilt auch für dreidimensionale Gebilde ausgehend von anderen Zell- oder Gewebetypen, beispielsweise zugänglichem erkranktem Patientengewebe wie Tumorgewebe. Vor allem eine räumlich korrekte Anordnung von Zelltypen (entlang von Gewebe-, Organ-, und Körperachsen), eine ausreichende Differenziertheit sowie eine ausreichende Größe der zellulären Gebilde im Millimeter- bis Zentimeterbereich sind mit den derzeit verfügbaren Verfahren und Vorrichtungen nicht erreichbar.

Die Dokumente US 2018/0030409 A1, WO 2017/083705 A1 und WO 2014/090993 A1 offenbaren beispielsweise Verfahren zum Entwickeln dreidimensionaler Organoide, bei denen die Versorgung von Zellkulturen mit Nährmedien mittels eines rotierenden Bioreaktors realisiert wird. Hier ist die Position des Organoids bzw. der im Organoid enthaltenen Zellen relativ zu Inhaltsstoffen des Nährmediums nicht fixiert. Die Verwendung solcher rotierenden Bioreaktoren hat den Nachteil, dass sich einzelne Gewebe- oder Organareale zufällig angeordnet und ungesteuert entwickeln, was zu einer für Forschungszwecke unzureichenden Qualität der entwickelten Organoide führt.

Ferner beschreibt das Dokument WO 2017/123791 A1 eine statische *in vitro* Entwicklung von dreidimensionalen Organoiden, wobei sich die Zellen ebenfalls zufällig positioniert und ungesteuert ausbilden.

Des Weiteren ist aus dem Dokument US 2015/0298123 A1 eine Plattform mit Mikrofluidiksystemen zur Kultivierung von Organoiden bekannt. Jedoch ist die Dauer der Kultivierung und somit die quantitative Entwicklung der Organoide aufgrund der geringen Dimensionen der Mikrofluidiksysteme begrenzt, d.h. den Organoiden steht ab einer bestimmten Größe nicht mehr genügend Platz für die weitere Entwicklung zur Verfügung.

Es ist daher eine Aufgabe der vorliegenden Erfindung, die Nachteile aus dem Stand der Technik zu überwinden. Insbesondere besteht eine Aufgabe der Erfindung darin, eine Vorrichtung, ein System und ein Verfahren bereitzustellen, mittels welcher/welchem ein zelluläres Ausgangsmaterial (ein/e oder mehrere Zelle/n, Zellgruppe/n oder Gewebe) in reproduzierbarer Weise zu einem dreidimensionalen zellulären Gebilde (Organ, Organoid, Gewebe, oder Zellgruppe) entwickelt und/oder aufrecht erhalten werden kann, so dass dieses Gebilde ausreichend räumlich organisiert, ausreichend ausdifferenziert und ausreichend groß dimensioniert ist. Im Sinne der vorliegenden Erfindung bedeutet dies insbesondere, dass die dreidimensionalen zellulären Gebilde einen Grad an Ausdifferenziertheit, Größe und/oder Organisiertheit erreicht haben, der dem des natürlichen Organs in einer Art und Weise nahekommt, dass bei der Verwendung im Krankheitsmodell besonders aussagekräftige Ergebnisse erzielt werden können. Dieselbe Ausdifferenziertheit, Größe und/oder Organisiertheit zu erreichen, die ein natürliches Organ aufweist, ist naturgemäß äußerst schwierig. In aller Regel ist dies jedoch auch nicht notwendig. Bereits ab einem gewissen Grad an Ausdifferenziertheit, Größe und/oder Organisiertheit können sehr aussagekräftige Ergebnisse erzielt werden, insbesondere Ergebnisse, die die im Stand der Technik erreichbaren Ergebnisse übertreffen. Bisherige Organoide aus dem Stand der Technik sind nicht ausreichend gut differenziert, groß und/oder organisiert. Im Falle von Gehirngewebe heißt das beispielsweise, dass sich einzelne Areale nur zufällig, nicht ausreichend differenziert, ungenau und/oder falsch angeordnet in Vorder-, Mittel- und Hinterhirn entwickeln.

Die Aufgaben werden durch eine Fluidikvorrichtung, ein Fluidiksystem und ein Verfahren gemäß den unabhängigen Patentansprüchen gelöst. Weiterbildungen und Ausführungsformen der Fluidikvorrichtung, des Fluidiksystems und des Verfahrens sind Gegenstand der abhängigen Ansprüche und der nachstehenden Beschreibung.

### Beschreibung der Erfindung

Die erfindungsgemäße Fluidikvorrichtung zum Entwickeln eines zellulären Ausgangsmaterials (ein/e) oder mehrere Zelle/n, Zellgruppe/n, Gewebe) zu einem oder mehreren dreidimensionalen zellulären Gebilde/n sowie zur Weiterentwicklung, verbesserten Reifung, Förderung des Größenwachstums und/oder Aufrechterhaltung dreidimensionaler zellulärer Gebilde umfasst einen Grundkörper mit einer sich in einer x-Richtung, einer zu der x-Richtung orthogonalen y-Richtung und einer zu der x-Richtung und der y-Richtung orthogonalen z-Richtung erstreckenden Kammer, in der eine Matrix (beispielsweise ein Hydrogel umfassend) aufgenommen oder aufnehmbar ist, z.B. derart, dass die Matrix die Kammer im Wesentlichen vollständig ausfüllt. Die Matrix dient u.a. als Gerüst, um eine definierte Position und Ausrichtung der Gebilde innerhalb der Kammer zu gewährleisten. Gleichzeitig kann sie das Wachstum, die Reifung und die Aufrechterhaltung der Gebilde beeinflussen. Aufrechterhaltung bedeutet insbesondere die Verhinderung von Zelltod durch permanente Mediumzufuhr. Die Kammer und somit die darin aufnehmbare oder aufgenommene Matrix können sich vorzugsweise mehrere hundert Mikrometer, weiter bevorzugt wenigstens einen Millimeter, weiter bevorzugt mehrere Millimeter, weiter bevorzugt wenigstens 3 mm, weiter bevorzugt wenigstens 4 mm, weiter bevorzugt wenigstens 6 mm, noch weiter bevorzugt wenigstens 9 mm, noch weiter bevorzugt mehrere Zentimeter, noch weiter bevorzugt wenigstens 5 cm, noch weiter bevorzugt wenigstens 7 cm, noch weiter bevorzugt wenigstens 10 cm in jede der drei Richtungen (x-, y-, z-) erstrecken, wobei der Betrag der Erstreckung in den drei Richtungen gleich oder unterschiedlich sein kann.

Der Grundkörper der erfindungsgemäßen Fluidikvorrichtung umfasst ferner wenigstens zwei Fluidreservoire, die vorzugsweise fluidisch voneinander getrennt sind. Fluidisch voneinander getrennt bedeutet insbesondere, dass die Fluidreservoire jeweils als separate Kavitäten ausgebildet sind und kein direkter Fluidaustausch zwischen den Fluidreservoiren stattfindet. In diesem Fall wäre allenfalls ein indirekter Fluidaustausch über die Kammer bzw. die Matrix möglich. Insbesondere kann der Grundkörper wenigstens vier Fluidreservoire umfassen.

Jedes der wenigstens zwei Fluidreservoire umfasst einen Fluideinlass zum vorzugsweise kontinuierlichen Einbringen eines Fluidmediums in das zugehörige Fluidreservoir, einen Fluidauslass zum vorzugsweise kontinuierlichen Auslassen des Fluidmediums aus dem zugehörigen Fluidreservoir, und eine für das Fluidmedium teildurchlässige Trenneinrichtung. Die Trenneinrichtung trennt das jeweils zugehörige Fluidreservoir von der Kammer und bildet eine gemeinsame flächige Schnittstelle des zugehörigen Fluidreservoirs mit der Kammer, über die das Fluidmedium in die Matrix eindiffundieren kann. Mit anderen Worten umfasst der Grundkörper somit wenigstens vier separate Fluideinlässe, wenigstens vier separate Fluidauslässe und wenigstens vier separate Trenneinrichtungen. Da jedes der wenigstens vier Fluidreservoire einen eigenen Fluideinlass aufweist, können die Fluidreservoire erfindungsgemäß mit unterschiedlichen Fluidmedien durchströmt werden.

Die erfindungsgemäße Fluidikvorrichtung ist dazu eingerichtet, bei Verwendung geeigneter Fluidmedien wenigstens einen Konzentrationsgradienten, wenigstens zwei zueinander orthogonale und/oder wenigstens zwei zueinander antiparallele Konzentrationsgradienten in der Matrix auszubilden, die jeweils über zumindest einen Abschnitt der Erstreckung der Matrix in der z-Richtung hinweg im Wesentlichen homogen oder gezielt inhomogen sind. Vorzugsweise sind der wenigstens eine Konzentrationsgradient, die wenigstens zwei zueinander orthogonalen und/oder die wenigstens zwei zueinander antiparallelen Konzentrationsgradienten in der Matrix über die nahezu gesamte Erstreckung der Matrix in der z-Richtung hinweg im Wesentlichen homogen.

Der Konzentrationsgradient bzw. die Konzentrationsgradienten können insbesondere Gradienten der Konzentration von Substanzen sein (beispielsweise von Morphogenen oder von Molekülen, welche morphogene Signalwege oder Differenzierungssignalwege beeinflussen). Konzentrationsgradienten im Sinne der vorliegenden Erfindung können stetig oder nichtstetig sein. So kann der Begriff Konzentrationsgradient in bestimmten Ausführungsformen auch komplizierte nicht-monotone Konzentrationsprofile umfassen. Beispielsweise kann der Begriff Konzentrationsgradient auch nicht-stabile Konzentrationsgradienten mit temporär hohen Konzentrationen in der Matrix umfassen, welche durch impulsartige Druckbeaufschlagung der Fluidreservoire ausgebildet werden können. Es versteht sich somit, dass geeignete Fluidmedien solche Fluidmedien sind, die ein Ausbilden eines Konzentrationsgradienten grundsätzlich ermöglichen.

Die zueinander orthogonalen und/oder antiparallelen Konzentrationsgradienten können mittels der erfindungsgemäßen Fluidikvorrichtung aufgrund der spezifischen strukturellen Ausgestaltung und Anordnung der Trenneinrichtungen untereinander sowie im Zusammenhang mit der Kammer und den Fluidreservoiren ausgebildet werden. Die erfindungsgemäße Fluidikvorrichtung ermöglicht somit das Ausbilden von definierten Konzentrationen biologischer oder nichtbiologischer Substanzen in allen Dimensionen der Matrix. Die Begriffe orthogonal und antiparallel beziehen sich auf die Richtungsvektoren der Konzentrationsgradienten. Der Begriff antiparallel bezeichnet somit Konzentrationsgradienten, die einander entgegengesetzte Richtungen aufweisen.

Diese spezifische Ausgestaltung und Anordnung sieht vor, dass zwei der flächigen Trenneinrichtungen sich jeweils in der z-Richtung und der x-Richtung erstrecken, wobei diese zwei Trenneinrichtungen einander gegenüberliegend angeordnet und durch die Kammer in der y-Richtung voneinander beabstandet sind. Hierdurch kann bei einer Verwendung voneinander verschiedener Fluidmedien in den Fluidreservoiren der zwei in y-Richtung beabstandeten Trenneinrichtungen ein in der y-Richtung zwischen diesen zwei Trenneinrichtungen verlaufender Konzentrationsgradient oder zwei zueinander antiparallele in der y-Richtung zwischen diesen zwei Trenneinrichtungen verlaufende Konzentrationsgradienten gebildet werden. Die zwei der Trenneinrichtungen können sich vorzugsweise mehrere hundert Mikrometer, weiter bevorzugt wenigstens einen Millimeter, weiter bevorzugt mehrere Millimeter, weiter bevorzugt wenigstens 3 mm, weiter bevorzugt wenigstens 4 mm, weiter bevorzugt wenigstens 6 mm, noch weiter bevorzugt wenigstens 9 mm, noch weiter bevorzugt mehrere Zentimeter, noch weiter bevorzugt wenigstens 5 cm, noch weiter bevorzugt wenigstens 7 cm, noch weiter bevorzugt wenigstens 10 cm in der z-Richtung und der x-Richtungen erstrecken, wobei der Betrag der Erstreckung in den beiden Richtungen gleich oder unterschiedlich sein kann. Die zwei der Trenneinrichtungen können vorzugsweise mehrere hundert Mikrometer, weiter bevorzugt wenigstens einen Millimeter, weiter bevorzugt mehrere Millimeter, weiter bevorzugt wenigstens 3 mm, weiter bevorzugt wenigstens 4 mm, weiter bevorzugt wenigstens 6 mm, noch weiter bevorzugt wenigstens 9 mm, noch weiter bevorzugt mehrere Zentimeter, noch weiter bevorzugt wenigstens 5 cm, noch weiter bevorzugt wenigstens 7 cm, noch weiter bevorzugt wenigstens 10 cm in der y-Richtung voneinander beabstandet sein.

Ferner sieht diese spezifische Ausgestaltung und Anordnung vor, dass zusätzlich oder alternativ zwei weitere der Trenneinrichtungen sich jeweils in der z-Richtung und der y-Richtung erstrecken, wobei diese zwei weiteren Trenneinrichtungen einander gegenüberliegend angeordnet und durch die Kammer in der x-Richtung voneinander beabstandet sind. Hierdurch kann bei einer Verwendung voneinander verschiedener Fluidmedien in den Fluidreservoiren der zwei in x-Richtung beabstandeten Trenneinrichtungen ein in der x-Richtung zwischen diesen zwei Trenneinrichtungen verlaufender Konzentrationsgradient oder zwei zueinander antiparallele in der x-Richtung zwischen diesen zwei Trenneinrichtungen verlaufende Konzentrationsgradienten gebildet werden. Die zwei weiteren der Trenneinrichtungen können sich vorzugsweise mehrere hundert Mikrometer, weiter bevorzugt wenigstens einen Millimeter, weiter bevorzugt mehrere Millimeter, weiter bevorzugt wenigstens 3 mm, weiter bevorzugt wenigstens 4 mm, weiter bevorzugt wenigstens 6 mm, noch weiter bevorzugt wenigstens 9 mm, noch weiter bevorzugt mehrere Zentimeter, noch weiter bevorzugt wenigstens 5 cm, noch weiter bevorzugt wenigstens 7 cm, noch weiter bevorzugt wenigstens 10 cm in der z-Richtung und der y-Richtungen erstrecken, wobei der Betrag der Erstreckung in den beiden Richtungen gleich oder unterschiedlich sein kann. Die zwei weiteren der Trenneinrichtungen können vorzugsweise mehrere hundert Mikrometer, weiter bevorzugt wenigstens einen Millimeter, weiter bevorzugt mehrere Millimeter, weiter bevorzugt wenigstens 3 mm, weiter bevorzugt wenigstens 4 mm, weiter bevorzugt wenigstens 6 mm, noch weiter bevorzugt wenigstens 9 mm, noch weiter bevorzugt mehrere Zentimeter, noch weiter bevorzugt wenigstens 5 cm, noch weiter bevorzugt wenigstens 7 cm, noch weiter bevorzugt wenigstens 10 cm in der x-Richtung voneinander beabstandet sein.

Eine flächige Schnittstelle ist demnach bevorzugt eine sich in der z-Richtung und in wenigstens einer dazu orthogonalen weiteren Raumrichtung (der x-Richtung oder der y-Richtung) erstreckende Schnittstelle.

Jede der Trenneinrichtungen kann sich vorzugsweise ausschließlich in den vorstehend beschriebenen Richtungen erstrecken, das heißt beispielweise nur in der z-Richtung und der x-Richtung oder nur in der z-Richtung und der y-Richtung. Sie können mit anderen Worten eine z-x-Ebene oder eine z-y-Ebene aufspannen. Bei einer Ausbildung von Trenneinrichtungen mit gleichen Dimensionen, die sich ausschließlich in den vorstehend beschriebenen Richtungen erstrecken (also nur in der z-Richtung und der x-Richtung bzw. nur in der z-Richtung und der y-Richtung), stellt die Anordnung und Ausbildung der vorstehend beschriebenen zwei weiteren der Trenneinrichtungen mit anderen Worten eine um 90 Grad um eine in der z-Richtung verlaufenden Achse gedrehte Anordnung und Ausbildung der zwei der Trenneinrichtungen dar.

Insbesondere kann das eine der Fluidreservoire einander gegenüberliegender Trenneinrichtungen mit einem Fluidmedium in Form eines Zellkulturmediums mit einer oder mehreren zusätzlichen biologischen oder nicht-biologischen Substanzen (beispielswiese Morphogene, morphogene Signalwege oder Differenzierungssignalwege beeinflussende Moleküle, Nährstoffe, Wachstumsfaktoren, Medikamente) durchströmt werden, während das andere Fluidreservoir mit einem Fluidmedium in Form entweder eines Zellkulturmediums ohne diese zusätzlichen Substanzen (zur Bildung eines dazwischen verlaufenden Konzentrationsgradienten) oder eines Zellkulturmediums mit anderen Substanzen (zur Bildung zweier dazwischen verlaufender antiparalleler Konzentrationsgradienten) durchströmt wird. Somit kann durch die strukturelle Anordnung und Ausbildung der Komponenten der Fluidikvorrichtung an einer Seite der Matrix ein Zellkulturmedium mit einer Substanz (z.B. Morphogen) über eine der Trennwände in die Matrix eindiffundieren und an einer entgegengesetzten Seite der Matrix ein Zellkulturmedium ohne oder mit einer anderen Substanz (z.B. Morphogen) über eine andere der Trennwände in die Matrix eindiffundieren, sodass sich zwischen entgegengesetzten Seiten der Matrix jeweils ein Konzentrationsgradient oder zwei antiparallele Konzentrationsgradienten über die Zeit ausbilden. Bei einer Verwendung von Fluidmedien mit Medikamenten kann die erfindungsgemäße Vorrichtung für Medikamententests mit dreidimensionalen zellulären Gebilden genutzt werden, um die Konzentrationsabhängigkeit von Zelleffekten zu messen.

Da die Kammer bzw. die darin aufgenommene oder aufnehmbare Matrix mehrere Paare (wenigstens zwei Paare) einander entgegengesetzter Seiten hat, die einander benachbart sind, können mittels der erfindungsgemäßen Fluidikvorrichtung zusätzlich oder alternativ zu den antiparallelen Konzentrationsgradienten zueinander orthogonale Konzentrationsgradienten in der Matrix ausgebildet werden. Die beiden Zellkulturmedien mit Substanzen können sich voneinander unterscheiden.

Es versteht sich, dass je nach Anwendungsfall bzw. je nach verwendeten Fluidmedien mit der erfindungsgemäßen Fluidikvorrichtung wahlweise wenigstens ein Konzentrationsgradient, wenigstens zwei Konzentrationsgradienten (zwei orthogonale Konzentrationsgradienten oder zwei antiparallele Konzentrationsgradienten), wenigstens drei Konzentrationsgradienten (zwei zueinander antiparallele Konzentrationsgradienten und ein zu diesen orthogonaler Konzentrationsgradient) oder wenigstens vier Konzentrationsgradienten (zwei zueinander antiparallele Konzentrationsgradienten und zwei zu diesen orthogonale und zueinander antiparallele Konzentrationsgradienten) ausgebildet werden können. Grundsätzlich kann die erfindungsgemäße Fluidikvorrichtung aufgrund ihrer strukturellen Konfiguration geeignet sein, wenigstens zwei Konzentrationsgradienten in der Matrix auszubilden. Es versteht sich, dass die Fluidikvorrichtung zum Ausbilden von lediglich einem Konzentrationsgradienten oder von zwei zueinander antiparallelen Konzentrationsgradienten lediglich zwei Fluidreservoire mit zugehörigen (d.h. zwei einander gegenüberliegenden) Trenneinrichtungen umfassen muss. Auch versteht sich, dass die Fluidikvorrichtung zum Ausbilden von zwei zueinander orthogonalen, von drei oder vier Konzentrationsgradienten vier Fluidreservoire mit zugehörigen (d.h. zwei Paaren einander jeweils gegenüberliegender) Trenneinrichtungen umfassen muss.

Eine Homogenität des/der Konzentrationsgradienten über zumindest einen Abschnitt der Erstreckung, vorzugsweise über die gesamte Erstreckung, der Matrix in der z-Richtung hinweg wird über die strukturelle Ausbildung der Fluidreservoire im Zusammenhang mit den damit in struktureller oder funktioneller Verbindung stehenden Komponenten der Fluidikvorrichtung erreicht, wie insbesondere den zugehörigen Trenneinrichtungen, der Kammer sowie den Fluideinlässen und Fluidauslässen. Durch die jeweils zugehörigen Fluideinlässe und Fluidauslässe können die Fluidreservoire kontinuierlich durchströmt und somit kontinuierlich mit frischen Fluidmedien versorgt werden. Es kann somit ein kontinuierlicher Austausch von frischem und altem Fluidmedium vorgesehen werden, um eine optimale Nährstoffversorgung zu erreichen und Abfallstoffe abzuführen. Gleichzeitig sorgen die Fluidreservoire dafür, dass sich das einströmende Fluidmedium in einem vorbestimmten Maß in dem Fluidreservoir staut, sodass stets die gesamte teildurchlässige Trenneinrichtung auf einer dem zugehörigen Fluidreservoir zugewandten Seite vollständig, d.h. über die gesamte Fläche der Trenneinrichtung, mit Fluidmedium bedeckt ist. Hierdurch kann das Volumen und die Konzentration des über jede der Trenneinrichtungen in die Matrix eindiffundierenden Fluidmediums über die gesamte z-Erstreckung der jeweiligen Trenneinrichtung konstant gehalten werden, wodurch sich über zumindest einen Abschnitt der Erstreckung, vorzugsweise über die gesamte Erstreckung, der Matrix in der z-Richtung hinweg, ein homogener Konzentrationsgradient in der Matrix ausbildet.

Ein homogener Konzentrationsgradient in der z-Richtung bedeutet bevorzugt, dass das Verhältnis der Konzentration von Substanzen (z.B. Morphogenen) an einer ersten Position z₁ und an einer zweiten Position z₂ in einem Bereich von 0,8:1 bis 1,2:1, weiter bevorzugt von 0,9:1 bis 1,1:1, weiter bevorzugt von 0,95:1 bis 1,05:1 liegt, wobei sich Position z₁ von Position z₂ lediglich in Bezug auf die z-Richtung, nicht jedoch in Bezug auf die x-Richtung und die y-Richtung unterscheidet. Insbesondere gelten die genannten Verhältnisse der Substanzkonzentrationen bevorzugt für sämtliche derartige Positionen zₙ und zₘ über die gesamte z-Erstreckung. In anderen Worten bedeutet ein homogener Konzentrationsgradient in der z-Richtung bevorzugt, dass sich der Gradient in verschiedenen durch die x-Richtung und die y-Richtung aufgespannten Ebenen entlang der z-Erstreckung nicht oder nur unwesentlich, insbesondere im Rahmen der oben genannten Verhältnisse der Substanzkonzentrationen, unterscheidet.

Ein inhomogener Konzentrationsgradient bedeutet demgegenüber bevorzugt, dass das Verhältnis der Konzentration von Substanzen (z.B. Morphogenen) an einer ersten Position z₁ und an einer zweiten Position z₂ kleiner als 0,8:1 oder größer als 1,2:1 ist, wobei sich Position z₁ von Position z₂ lediglich in Bezug auf die z-Richtung, nicht jedoch in Bezug auf die x-Richtung und die y-Richtung unterscheidet. Insbesondere gelten die genannten Verhältnisse der Substanzkonzentrationen bevorzugt für sämtliche derartige Positionen zₙ und zₘ über die gesamte z-Erstreckung. In anderen Worten bedeutet ein inhomogener Konzentrationsgradient in der z-Richtung bevorzugt, dass sich der Gradient in verschiedenen durch die x-Richtung und die y-Richtung aufgespannten Ebenen entlang der z-Erstreckung im Rahmen der oben genannten Verhältnisse der Substanzkonzentrationen unterscheidet.

Eine vorbestimmte (gezielte) Inhomogenität des/der Konzentrationsgradienten kann beispielsweise erreicht werden, indem eine oder jede der Trenneinrichtungen unterschiedliche Teildurchlässigkeiten innerhalb der Trenneinrichtung aufweisen. So kann z.B. im Zentrum der Kammer eine höhere Konzentrationsdifferenz zwischen zwei gegenüberliegenden Seiten eingestellt werden als in lateralen Bereichen der Kammer. Eine gezielte Inhomogenität unterscheidet sich von einer nichtgezielten (zufälligen) Inhomogenität dadurch, dass die erstgenannte über entsprechende Maßnahmen in definierter Weise und reproduzierbar einstellbar ist. In weiteren Ausführungsformen kann zu der oben beschriebenen Homogenität oder gezielten Inhomogenität des Konzentrationsgradienten in z-Richtung eine zusätzliche Homogenität oder zusätzliche gezielte Inhomogenität in einer Raumrichtung eingestellt werden, die sowohl zur z-Richtung als auch zur Richtung des Konzentrationsgradienten orthogonal ist. Die vorstehenden Ausführungen zur Homogenität und zur gezielten Inhomogenität des Konzentrationsgradienten in z-Richtung gelten für diese zusätzliche Homogenität oder zusätzliche gezielte Inhomogenität über die genannte Raumrichtung entsprechend.

Durch das Ausbilden wenigstens zweier zueinander orthogonaler und/oder antiparalleler Konzentrationsgradienten in der das zelluläre Ausgangsmaterial enthaltenden Matrix, die jeweils über eine Erstreckung der Matrix in der z-Richtung hinweg homogen oder gezielt inhomogen sind, kann die dreidimensionale Entwicklung des zellulären Ausgangsmaterials zu dem dreidimensionalen zellulären Gebilde (z.B. einer Zellkultur zu einem Organoid) räumlich gesteuert werden. Damit ermöglicht die erfindungsgemäße Fluidikvorrichtung eine gezielte gleichzeitige Entwicklung mehrerer Gewebeareale, z.B. dorsales und ventrales Vorder-, Mittel- und Hinterhirn. Es versteht sich, dass neben Gehirngewebe auch anderes Organgewebe mittels der erfindungsgemäßen Fluidikvorrichtung entwickelt werden kann. Zudem erhöht die Verwendung der erfindungsgemäßen Fluidikvorrichtung im Vergleich zu bekannten Systemen die Reproduzierbarkeit entwickelter Organoide, da die Konzentrationsgradienten mittels der Fluidikvorrichtung in räumlicher Hinsicht und in ihrer Intensität gezielt in der Matrix ausgebildet werden können.

Darüber hinaus kann durch die Erstreckung der Kammer und der darin aufgenommenen oder aufnehmbaren Matrix in drei verschiedene Raumrichtungen ein dreidimensionales zelluläres Gebilde in größeren Dimensionen entwickelt werden, als dies mit bisherigen Vorrichtungen möglich ist. So ist mit bestimmten Ausführungsformen der erfindungsgemäßen Fluidikeinrichtung die Entwicklung von zellulären Gebilden im Millimeterbereich bis hin zum Zentimeter-Bereich realisierbar.

Gegenüber Vorrichtungen des Standes der Technik hat die erfindungsgemäße Fluidikvorrichtung ferner den Vorteil, dass über ein gesteuertes Einbringen und Auslassen von Fluidmedien in die bzw. aus den Fluidreservoiren der Druck in den einzelnen Fluidreservoiren und somit an verschiedenen Seiten der Matrix geregelt werden kann. Hierdurch können mittels Druckbeaufschlagung gezielt frische Nährstoffe in das Innere von vergleichsweise großen zellulären Gebilden eingebracht und/oder mittels Druckabbau ein Abfließen von Stoffwechselabfallprodukten begünstigt werden. Diese Effekte können für ein Größenwachstum und/oder die Aufrechterhaltung von großen Organoiden und anderen zellulären Gebilden sehr wichtig sein und zusätzliche Anwendungen der Fluidikvorrichtung erschließen. Dies ist mittels bekannter Vorrichtungen des Standes der Technik nicht realisierbar, weshalb bei diesen ab einer gewissen Organoid-Größe (typischerweise im geringen mm-Bereich) Zellen im Inneren des Organoids nur noch schlecht versorgt werden und absterben, da der Diffusionsweg von Inhaltsstoffen des Nährmediums ins Organoidinnere zu groß wird und der Transport von Nährmediuminhaltsstoffen außerdem nicht durch Erhöhung des Drucks im das Organoid umgebende Nährmedium verbessert werden kann. Auch das erzielbare Größenwachstum ist aufgrund der mangelnden Versorgung mittels bekannter Vorrichtungen begrenzt.

Eine Ausführungsform betrifft eine Fluidikvorrichtung umfassend einen Grundkörper mit einer sich in einer x-Richtung, einer zu der x-Richtung orthogonalen y-Richtung und einer zu der x-Richtung und der y-Richtung orthogonalen z-Richtung erstreckenden Kammer, in der eine Matrix aufgenommen oder aufnehmbar ist, in die das zu entwickelnde Ausgangsmaterial einbringbar ist; und wenigstens vier Fluidreservoiren. Jedes der wenigstens vier Fluidreservoire umfasst einen Fluideinlass zum Einbringen eines Fluidmediums in das zugehörige Fluidreservoir, einen Fluidauslass zum Auslassen des Fluidmediums aus dem zugehörigen Fluidreservoir, und eine für das Fluidmedium teildurchlässige Trenneinrichtung, die das zugehörige Fluidreservoir von der Kammer trennt und eine gemeinsame flächige Schnittstelle des zugehörigen Fluidreservoirs mit der Kammer bildet, über die das Fluidmedium in die Matrix eindiffundieren kann. Die Fluidikvorrichtung ist dazu eingerichtet, bei Verwendung geeigneter Fluidmedien wenigstens zwei zueinander orthogonale und/oder antiparallele Konzentrationsgradienten in der Matrix auszubilden, die jeweils über zumindest einen Abschnitt der Erstreckung der Matrix in der z-Richtung hinweg im Wesentlichen homogen sind, indem zwei der Trenneinrichtungen sich jeweils in der z-Richtung und der x-Richtung erstrecken und einander gegenüberliegend angeordnet und durch die Kammer in der y-Richtung voneinander beabstandet sind, und indem zwei weitere der Trenneinrichtungen sich jeweils in der z-Richtung und der y-Richtung erstrecken und einander gegenüberliegend angeordnet und durch die Kammer in der x-Richtung voneinander beabstandet sind.

In einer Ausführungsform der Fluidikvorrichtung kann die Kammer einen rechteckigen, vorzugsweise einen quadratischen, Querschnitt aufweisen. Demnach kann auch die darin aufgenommene oder aufnehmbare Matrix einen entsprechenden rechteckigen, vorzugsweise einen quadratischen, Querschnitt aufweisen.

In einer Weiterbildung können die Trenneinrichtungen der Fluidikvorrichtung miteinander verbunden sein und gemeinsam die zwischen ihnen liegende Kammer bilden. Genauer gesagt können benachbarte Trenneinrichtungen eine gemeinsame Kante aufweisen, die sich in der z-Richtung erstreckt. Die dazwischenliegende Kammer kann bei dieser Weiterbildung von den Trenneinrichtungen begrenzt und somit in ihrer Form und Erstreckung definiert sein. In diesem Fall können einander gegenüberliegende Trenneinrichtungen durch zwischen ihnen liegende Trenneinrichtungen mit einer anderen Orientierung in x-Richtung bzw. in y-Richtung voneinander beabstandet sein.

Gemäß einer Ausführungsform der Fluidikvorrichtung kann jede der Trenneinrichtungen in Form einer mit einer Vielzahl an Durchgangsöffnungen versehenen Trennwand ausgebildet sein. Die Durchgangsöffnungen können über die gesamte Trennwand gleichmäßig und/oder nach einem bestimmten Muster verteilt sein. Durch ungleichmäßig verteilte und/oder ungleichmäßig große Öffnungen in einer Trennwand kann ein inhomogener Konzentrationsgradient gezielt generiert werden, welcher beispielsweise über die Erstreckung der Matrix in der z-Richtung hinweg unterschiedlich ist und somit verschiedene Areale des Gebildes entlang der z-Richtung unterschiedlich beeinflusst. Entsprechend kann ein inhomogener Konzentrationsgradient, d.h. eine unterschiedliche Konzentrationsdifferenz, über die Erstreckung der Matrix in der x-Richtung und/oder in der y-Richtung gezielt ausgebildet werden. Insbesondere kann hierzu die Anzahl der Durchgangsöffnungen in der entsprechenden Richtung variiert werden. Ein Ausbilden eines oder mehrerer inhomogener Konzentrationsgradienten ermöglicht, in einem dreidimensionalen zellulären Gebilde verschiedene Effekte an verschiedenen Positionen hervorzurufen.

Die Trennwände können jeweils mit 0,5 bis 50, vorzugsweise mit 0,5 bis 5, bevorzugt mit 1,5 bis 4, weiter bevorzugt mit 2 bis 3,5, noch weiter bevorzugt mit 2,5 bis 3 Durchgangsöffnungen pro mm² versehen sein. Die Durchgangsöffnungen können insbesondere einen kreisförmigen Querschnitt haben, mit einem Durchmesser zwischen 0,1 mm und 1,5 mm, vorzugsweise 0,2 mm und 1,2 mm, weiter vorzugsweise zwischen 0,4 mm und 1,0 mm, weiter bevorzugt zwischen 0,5 mm und 0,8 mm. Die Durchgangsöffnungen innerhalb einer Trennwand und zwischen den verschiedenen Trennwänden können die gleiche Querschnittsfläche oder unterschiedliche Querschnittsflächen haben. Auch können die Trennwände jeweils die gleiche Anzahl oder unterschiedliche Anzahlen an Durchgangsöffnungen aufweisen. Derart perforierte Seitenwände dienen dazu das System stabil zu halten und Fluidmedium in vorgegebenem Maße passieren zu lassen. Die Dicke der Trennwände ist so gewählt, dass die mechanische Stabilität der Fluidikvorrichtung gewährleistet ist. Gleichzeitig sind möglichst dünne Trennwände gewünscht, damit die Fluidmedien mit Substanzen möglichst ungehindert durch die perforierten Wände diffundieren können. Hierzu können die Trennwände eine Dicke zwischen 0,05 mm und 1,5 mm, vorzugsweise zwischen 0,1 mm und 1 mm aufweisen. Die Dicke der Trennwände sowie die Anzahl, Größe, Geometrie und Anordnung der Durchgangsöffnungen kann so gewählt werden, dass die Trenneinrichtungen zwar für die verwendeten Fluidmedien teildurchlässig sind, aber gleichzeitig ein Durchtreten der Matrix durch die Durchgangsöffnungen verhindert wird.

Alternativ zu Trenneinrichtungen in Form von Trennwänden können in anderen Ausführungsformen Trenneinrichtungen in Form von Membranen, Gitteranordnungen oder Ähnlichem ausgebildet sein.

In einer Weiterbildung der Fluidikvorrichtung kann die Kammer eine Eingangsöffnung und eine Ausgangsöffnung umfassen, zwischen denen sich die Kammer in der z-Richtung erstreckt. Die Eingangsöffnung und die Ausgangsöffnung sind in z-Richtung betrachtet kongruent zueinander oder überlappen sich zumindest, sodass die Kammer mittels optischer Strahlen in der z-Richtung durchleuchtbar ist. Hierdurch kann beispielsweise eine kontinuierliche Analyse der differenzierenden Organoide während der Entwicklung ermöglicht werden, da die Kammer in der Mitte durchgängig und über die Eingangsöffnung und die Ausgangsöffnung nach oben bzw. nach unten hin geöffnet ist. Insbesondere ermöglicht die Weiterbildung der Kammer mit der Eingangsöffnung und der Ausgangsöffnung die Verwendung eines Mikroskops zum Beobachten und Analysieren des zellulären Ausgangsmaterials bzw. des dreidimensionalen zellulären Gebildes (z.B. der Zellkultur bzw. des Organoids) während des Entwickelns. Zudem stellt die Weiterbildung mit einer Eingangsöffnung und einer Ausgangsöffnung ein offenes System dar, bei dem die Matrix in der Kammer mit Umgebungsluft im Gasaustausch stehen kann.

Die vorstehend beschriebene Weiterbildung macht die Fluidikvorrichtung zwar mikroskopierbar, beschränkt jedoch die Anzahl der in der Matrix ausbildbaren orthogonalen und/oder antiparallelen Konzentrationsgradienten auf insgesamt maximal vier verschiedene Konzentrationsgradienten (zwei zueinander antiparallele x-Konzentrationsgradienten, die sich entlang der x-Richtung erstrecken, sowie zwei zu diesen orthogonale und zueinander antiparallele y-Konzentrationsgradienten, die sich entlang der y-Richtung erstrecken). Dies ist jedoch für nahezu alle praxisrelevanten Anwendungsfälle ausreichend, da die meisten Gewebe/Organe sich entlang zweier Hauptachsen entwickeln (anterior-posterior [vorne-hinten] und dorsal-ventral [oben-unten]). Jedoch kann in einer alternativen Weiterbildung der Fluidikvorrichtung auch die Ausbildung von insgesamt sechs Konzentrationsgradienten in der Matrix ermöglicht werden (zwei zueinander antiparallele x-Konzentrationsgradienten, die sich entlang der x-Richtung erstrecken, zwei zu diesen orthogonale und zueinander antiparallele y-Konzentrationsgradienten, die sich entlang der y-Richtung erstrecken, sowie zwei zu diesen orthogonale und zueinander antiparallele z-Konzentrationsgradienten, die sich entlang der z-Richtung erstrecken). In diesem Fall kann die Entwicklung entlang der anterior-posterioren Achse, der dorsal-ventralen Achse und der rechtslinks-Achse gesteuert werden. In einer solchen alternativen Weiterbildung umfasst die Fluidikvorrichtung, genauer gesagt der Grundkörper, zwei zusätzliche der Trenneinrichtungen, die sich jeweils in der x-Richtung und der y-Richtung erstrecken, wobei diese zwei zusätzlichen Trenneinrichtungen einander gegenüberliegend angeordnet und durch die Kammer in der z-Richtung voneinander beabstandet sind. Hierdurch kann bei einer Verwendung voneinander verschiedener Fluidmedien in den zu diesen zwei in z-Richtung beabstandeten Trenneinrichtungen zugehörigen Fluidreservoiren ein zwischen diesen zwei Trenneinrichtungen liegender dritter Konzentrationsgradient gebildet werden.

In einer Ausführungsform kann die Fluidikvorrichtung ferner einen Deckel umfassen, der mit dem Grundkörper verbunden oder verbindbar ist und zumindest eines oder alle der wenigstens vier Fluidreservoire zu einer Seite hin in der z-Richtung dichtend verschließt. Der Deckel kann insbesondere lösbar mit dem Grundkörper verbunden oder verbindbar sein, beispielsweise mittels einer Schraubverbindung. Der Deckel kann sich zudem abschnittsweise über die Eingangsöffnung der Kammer hinweg erstrecken und diese zumindest teilweise in der z-Richtung verschließen, wobei der Deckel eine Deckelausnehmung aufweisen kann, die in der z-Richtung mit der Eingangsöffnung der Kammer ausgerichtet ist. Somit kann während der Entwicklung des dreidimensionalen zellulären Gebildes der Entwicklungsverlauf durch die Deckelausnehmung hindurch beobachtet, insbesondere mikroskopiert, werden. Indem die Fläche der Deckelausnehmung kleiner als die Fläche der Eingangsöffnung der Kammer gewählt wird, kann der Deckel ferner dazu dienen ein ungewünschtes Austreten der Matrix in der z-Richtung zu verhindern.

Die Fluidikvorrichtung kann ferner eine Bodenkomponente umfassen, die mit dem Grundkörper verbunden oder verbindbar ist und zumindest eines oder alle der wenigstens vier Fluidreservoire zu einer anderen Seite hin in der z-Richtung dichtend verschließt, wobei die Bodenkomponente an einer dem Deckel entgegengesetzten Seite der Kammer angeordnet ist. Die Bodenkomponente kann lösbar mit dem Grundkörper verbunden oder verbindbar sein, beispielsweise mittels einer Schraubverbindung. Zudem kann die Bodenkomponente sich abschnittsweise über die Ausgangsöffnung der Kammer hinweg erstrecken und diese zumindest teilweise in der z-Richtung verschließen, wobei die Bodenkomponente eine Bodenausnehmung aufweisen kann, die in der z-Richtung mit der Ausgangsöffnung der Kammer ausgerichtet ist. Somit kann während der Entwicklung des Organoids der Entwicklungsverlauf durch die Bodenausnehmung bzw. die Deckelausnehmung und die Bodenausnehmung hindurch beobachtet, insbesondere mikroskopiert, werden. Auch ist somit ein einfaches Einsetzen und eine Entnahme von Gebilden aus der Kammer möglich. Indem die Fläche der Bodenausnehmung kleiner als die Fläche der Ausgangsöffnung der Kammer gewählt wird, kann die Bodenkomponente ferner dazu dienen ein ungewünschtes Austreten der Matrix in der z-Richtung zu verhindern. Die Mikroskopierbarkeit der Fluidikvorrichtung kann weiter verbessert werden, indem die Bodenkomponente im Bereich der Bodenausnehmung vorzugsweise mit einem Deckglas versehen ist.

Wenigstens eines der Fluidreservoire kann offen ausgebildet sein, um eine Verbindung mit einem Inkubator zu ermöglichen.

Die Bodenkomponente kann in Form eines Kegelstumpfes oder eines Pyramidenstumpfes ausgebildet sein, dessen Deckfläche an den Grundkörper angrenzt. Hierdurch kann die Bodenkomponente als Fuß der Fluidikvorrichtung dienen und den Grundkörper stabil gegenüber einem Untergrund abstützen.

Die Bodenkomponente kann in einer Ausführungsform mit Ausnehmungen, Nuten, Rinnen oder Ähnlichem versehen sein. Diese können derart an der Bodenkomponente angeordnet und ausgebildet sein, dass in einem miteinander verbundenen Zustand des Grundkörpers und der Bodenkomponente jeweils eine der Ausnehmungen, Nuten, Rinnen oder Ähnlichem an jeweils einen der Fluidauslässe der Fluidreservoire angrenzt, um die aus den Fluidauslässen kontinuierlich austretenden Fluidmedien gezielt abzuführen, beispielsweise in einen oder mehrere benachbarte Auffangbehälter.

Der Grundkörper der Fluidikvorrichtung kann aus Metall, vorzugsweise aus Edelstahl, Glas, Keramik oder Polymer hergestellt sein. Auch die anderen Komponenten der Fluidikvorrichtung können aus einem dieser Materialien hergestellt sein, vorzugsweise aus demselben Material wie der Grundkörper. Insbesondere können die Komponenten der Fluidikvorrichtung aus einem Material hergestellt sein, das autoklavierbar, nicht zelltoxisch und nicht autofluoreszierend ist. Die Autoklavierbarkeit des verwendeten Materials ermöglicht die Wiederverwendbarkeit der Fluidikvorrichtung. Zudem sind zelltoxische Materialien zu vermeiden, da sie das Wachstum des zu entwickelnden zellulären Ausgangsmaterials nicht behindern. Der Einsatz nicht autofluoreszierender Materialien sorgt dafür, dass beim Mikroskopieren fluoreszierender Bestandteile keine ungewünschten Interferenzen entstehen.

Die Matrix dient dazu, das zu entwickelnde zelluläre Ausgangsmaterial bzw. das sich entwickelnde dreidimensionale zelluläre Gebilde während des Entwickelns in der vorgesehenen Position zu halten, vorzugsweise im Zentrum der Kammer. Auch können mehrere dreidimensionale zelluläre Gebilde in einer einzelnen Kammer untergebracht werden. Darüber hinaus erlaubt die Matrix das quantitative Wachstum und die Differenzierung des zellulären Ausgangsmaterials. Zudem lässt die Matrix die Diffusion von Fluidmedien zu. Vorzugsweise wird als Matrix optisch transparentes Material verwendet, um eine Beobachtung der Entwicklung des Ausgangsmaterials bzw. der Entwicklung dreidimensionaler zellulärer Gebilde über die Zeit zu ermöglichen. Die Matrix kann insbesondere eine Hydrogel-Matrix sein. Die Matrix kann insbesondere eine Agarose-Matrix sein. Alternativ dazu kann als Matrix beispielsweise eine KollagenMatrix, eine basalmembranartige Matrix (beispielsweise Matrigel^{®}), eine synthetische Matrix oder Ähnliches eingesetzt werden. Eine Matrix aus Agarose hat den Vorteil, dass sie aus definierten Komponenten besteht, deren Zusammensetzung nicht schwankt. Eine Agarose-Matrix erlaubt reproduzierbare Bedingungen und beeinflusst die Differenzierung und das Größenwachstum nicht negativ. Die Konzentration und somit die Diffussionseigenschaften einer Agarose-Matrix sind veränderbar. Eine Agarose-Matrix ist kostengünstiger als Matrigel^{®} und kann in der Zusammensetzung leicht standardisiert werden.

Bevorzugt liegt der Agarose-Anteil an der Matrix in einem Bereich von 0,4-1,2 Gew-%. Ein sehr hoher Agarose-Anteil ist nachteilig, da sonst das Größenwachstum des Organoids behindert sein kann. Der Agarose-Anteil sollte jedoch auch nicht sehr klein sein, da es ansonsten zu einer reduzierten Stabilität und Druckresistenz der Matrix kommen kann und die Matrix aus dem Inneren der Kammer fallen kann. Zudem sollte der Agarose-Anteil ausreichend hoch sein, um die Position des dreidimensionalen zellulären Gebildes stabil zu halten. Eine Kombination verschiedener Agarose-Konzentrationen im inneren Bereich der Kammer und am äußeren Bereich (zu den Trennwänden hin) ist möglich. Generell sind Kombinationen verschiedener Matrix-Substanzen möglich. In manchen Ausführungsformen umfasst die Matrix verschiedene Substanzen.

In einer Weiterbildung der erfindungsgemäßen Fluidikvorrichtung können der Fluideinlass und der Fluidauslass jedes Fluidreservoirs in der z-Richtung voneinander beabstandet und vorzugsweise auf einer sich in der z-Richtung erstreckenden Linie angeordnet sein. Mit anderen Worten können der Fluideinlass und der Fluidauslass jedes Fluidreservoirs eine vertikale Anordnung aufweisen. Dies ermöglicht eine optimale kontinuierliche Durchströmung jedes Fluidreservoirs. Die Durchströmung der Fluidreservoire kann weiter verbessert werden, indem in bestimmten Ausführungsformen der Fluidauslass jedes Fluidreservoirs eine größere Querschnittsfläche aufweist als der Fluideinlass desselben Fluidreservoirs. Alternativ können der Fluideinlass und der Fluidauslass jedes Fluidreservoirs eine horizontale oder sonstige Anordnung aufweisen. Der Fluideinlass und der Fluidauslass jedes Fluidreservoirs können beispielsweise in einem 24 Grad-Winkel zueinander stehen. Die Fluideinlässe und Fluidauslässe können jeweils zum Reservoir hin einen geringeren Durchmesser haben. Dies kann bei einem Anschluss von Schläuchen an die Fluideinlässe und/oder Fluidauslässe verhindern, dass ein vorderes Ende der Schläuche ungewünscht in das Reservoir gelangt.

Die Erfindung betrifft ferner ein Fluidiksystem zum Entwickeln eines zellulären Ausgangsmaterials zu einem oder mehreren dreidimensionalen zellulären Gebilden sowie zur Weiterentwicklung, verbesserten Reifung, Förderung des Größenwachstums und/oder Aufrechterhaltung dreidimensionaler zellulärer Gebilde. Das erfindungsgemäße Fluidiksystem umfasst eine Fluidikvorrichtung der vorstehend beschriebenen Art und ein Pumpensystem, bei dem jeder Fluideinlass über einen Einlassschlauch mit einer Pumpe zum vorzugsweise kontinuierlichen Einbringen eines Fluidmediums in das zugehörige Fluidreservoir verbunden ist. Das Einbringen von Fluidmedium in jedes Fluidreservoir kann somit über das Pumpensystem in vorbestimmter Weise gesteuert werden.

Jeder Pumpe kann dazu eingerichtet sein, das Fluidmedium mit einer Flussrate zwischen 1 und 1000 µl/h, vorzugsweise zwischen 1 und 100 µl/h, weiter bevorzugt zwischen 15 und 60 µl/h, weiter bevorzugt zwischen 20 und 50 µl/h durch den Fluideinlass zu fördern. Die Flussrate wird so gewählt, dass pro Zeiteinheit deutlich mehr Fluidmedium mit Substanz (z.B. Morphogen) einem Fluidreservoir zugeführt wird als Fluidmedium mit Substanz (z.B. Morphogen) durch Diffusion das Fluidreservoir verlässt, während gleichzeitig ein Zusammenpressen der Matrix durch die Fluidmedien vermieden wird. Abgesehen von konstanten Flussraten können die Konzentrationsgradienten auch durch dynamische Flussraten erreicht werden. Durch das Einstellen dynamischer Flussraten können die Fluidreservoire jeweils dynamisch, insbesondere pulsartig, mit Druck (Impulsen) beaufschlagt werden, wodurch in der Matrix Konzentrationsgradienten in Form von komplizierten nicht-monotonen Konzentrationsprofilen ausgebildet werden können.

Ferner können mittels des Pumpensystems definierte Druckdifferenzen zwischen gegenüberliegenden Fluidreservoiren hergestellt werden können. Dies kann für bestimmte Anwendungen relevant sein, insbesondere für Prozesse nach einer Entwicklung eines organisierten dreidimensionalen zellulären Gebildes, wie einem Aufrechterhalten von dreidimensionalen zellulären Gebilden oder einem gerichteten Abführen von Stoffwechselprodukten.

Das Pumpensystem kann insbesondere ein Spritzenpumpensystem sein. Die Pumpen sind in diesem Fall Spritzenpumpen.

In einer Weiterbildung des Fluidiksystems kann jeder Fluidauslass der Fluidikvorrichtung mit einem Auslassschlauch zum Auslassen des Fluidmediums aus dem zugehörigen Fluidreservoir verbunden sein. Jeder Auslassschlauch kann vorzugsweise mit einer Auslasspumpe zum Beaufschlagen des Fluidauslasses mit einem vorbestimmten Unterdruck verbunden sein. Hierdurch kann die Flussrate und somit die Durchströmung der Fluidreservoire mit Fluidmedium noch besser gesteuert werden. Es können Pumpen nur an die Einlassschläuche, nur an die Auslassschläuche oder an beide gleichzeitig angeschlossen werden.

In einer weiteren Ausführungsform des Fluidiksystems können wenigstens zwei, vorzugsweise eine Vielzahl von, Fluidikvorrichtungen der vorstehend beschriebenen Art vorgesehen sein, die in Form einer Parallelschaltung angeordnet und parallel mittels des Pumpensystems mit Fluidmedien versorgbar sind. Auch kann eine Parallelschaltung durch Unterdruckpumpsysteme an den Auslässen oder eine Parallelschaltung durch Kombination von Einlass- und Auslasspumpen realisiert werden.

Die Erfindung betrifft ferner ein Verfahren zum Entwickeln von zellulärem Ausgangsmaterial zu einem oder mehreren dreidimensionalen zellulären Gebilden und/oder zu deren Reifung, Grö-ßenwachstum oder Aufrechterhaltung, wobei das Verfahren die Schritte umfasst:
- Bereitstellen des zu entwickelnden zellulären Ausgangsmaterials in einer Matrix, die sich in einer x-Richtung, einer zu der x-Richtung orthogonalen y-Richtung und einer zu der x-Richtung und der y-Richtung orthogonalen z-Richtung erstreckt; und
- Ausbilden wenigstens eines Konzentrationsgradienten, wenigstens zweier zueinander orthogonaler Konzentrationsgradienten und/oder wenigstens zweier zueinander antiparalleler Konzentrationsgradienten in der Matrix, wobei jeder der Konzentrationsgradienten sich in der x-Richtung oder der y-Richtung erstreckt und ein in der z-Richtung homogener oder gezielt inhomogener Konzentrationsgradient ist. Genauer gesagt ist jeder der Konzentrationsgradienten über wenigstens einen Abschnitt der Erstreckung oder die gesamte Erstreckung der Matrix in der z-Richtung hinweg homogen oder gezielt inhomogen.

Die Konzentrationsgradienten können insbesondere Gradienten der Konzentration von Morphogenen, morphogene Signalwege oder Differenzierungswege beeinflussenden Substanzen oder Medikamenten sein. In manchen Ausführungsformen können die Konzentrationsgradienten insbesondere Gradienten der Konzentration von Morphogenen oder morphogene Signalwege oder Differenzierungswege beeinflussenden Substanzen sein. In manchen Ausführungsformen können die Konzentrationsgradienten insbesondere Gradienten der Konzentration von Medikamenten sein.

In einer Weiterbildung betrifft die Erfindung ferner ein Verfahren zum Entwickeln von zellulärem Ausgangsmaterial zu einem oder mehreren dreidimensionalen zellulären Gebilden und/oder zu deren Reifung, Größenwachstum oder Aufrechterhaltung, wobei das Verfahren die Schritte umfasst:
- Bereitstellen des zu entwickelnden Ausgangsmaterials (ein/e oder mehrere Zelle/n, Zellgruppe/n, oder Gewebe) in einer Matrix, die in einer Kammer einer Fluidikvorrichtung aufgenommen ist, wobei sich die Kammer und die Matrix in einer x-Richtung, einer zu der x-Richtung orthogonalen y-Richtung und einer zu der x-Richtung und der y-Richtung orthogonalen z-Richtung erstrecken, und wobei die Fluidikvorrichtung wenigstens zwei fluidisch voneinander getrennte Fluidreservoire aufweist, wobei jedes der wenigstens zwei Fluidreservoire eine für das Fluidmedium teildurchlässige Trenneinrichtung umfasst, die das zugehörige Fluidreservoir von der Kammer trennt und eine gemeinsame flächige Schnittstelle des zugehörigen Fluidreservoirs mit der Kammer bildet, und wobei jeweils zwei der Trenneinrichtungen an einander entgegengesetzten Seiten der Matrix angeordnet sind;
- Durchströmen der wenigstens zwei Fluidreservoire mit jeweils einem Fluidmedium;
- Ausbilden wenigstens eines Konzentrationsgradienten, wenigstes zweier zueinander orthogonaler Konzentrationsgradienten und/oder wenigstens zweier antiparalleler Konzentrationsgradienten in der Matrix, die jeweils über zumindest einen Abschnitt der Erstreckung der Matrix in der z-Richtung hinweg im Wesentlichen homogen oder gezielt inhomogen sind, indem über die teildurchlässigen Trenneinrichtungen der wenigstens zwei durchströmten Fluidreservoire das jeweils zugehörige Fluidmedium über den Abschnitt der Erstreckung der Matrix in der z-Richtung hinweg in die Matrix eindiffundiert, wobei jeweils an einander entgegengesetzten Seiten der Matrix voneinander verschiedene Fluidmedien in die Matrix eindiffundieren.

Das Verfahren kann insbesondere mittels einer Fluidikvorrichtung oder einem Fluidiksystem der vorstehend beschriebenen Art ausgeführt werden.

Der Gegenstand der Erfindung ist auf den Gegenstand der Ansprüche und nicht nur auf die zuvor beschriebenen Ausführungsformen und/oder Weiterbildungen beschränkt. Zudem versteht sich, dass obgleich einige der Ausführungsformen, Weiterbildungen und Merkmale vorstehend lediglich in Bezug auf die Fluidikvorrichtung oder das Fluidiksystem beschrieben wurden, diese Ausführungsformen, Weiterbildungen und Merkmale entsprechend für das erfindungsgemäße Verfahren gelten können. Gleichermaßen können Ausführungsformen, Weiterbildungen und Merkmale, die vorstehend lediglich in Bezug auf das Verfahren beschrieben wurden, entsprechend für die Fluidikvorrichtung und/oder das Fluidiksystem gelten.

### Kurzbeschreibung der Figuren

Ausführungsbeispiele der vorliegenden Erfindung werden nachstehend in Bezug auf die beiliegenden schematischen Figuren näher erläutert. Es stellen dar:
- Fig. 1: eine perspektivische Ansicht einer Fluidikvorrichtung gemäß einem Ausführungsbeispiel.
- Fig. 2: eine Draufsicht auf die Fluidikvorrichtung gemäß dem Ausführungsbeispiel aus Fig. 1.
- Fig. 3: eine Schnittdarstellung der Fluidikvorrichtung gemäß dem Ausführungsbeispiel aus den Figuren 1 und 2.
- Fig. 4: eine perspektivische Ansicht der Fluidikvorrichtung gemäß dem Ausführungsbeispiel aus Fig. 1 mit einem Deckel und einer ersten Bodenkomponente.
- Fig. 5: eine Schnittdarstellung der Fluidikvorrichtung mit dem Deckel und der ersten Bodenkomponente gemäß Fig. 4.
- Fig. 6: eine perspektivische Ansicht der Fluidikvorrichtung gemäß dem Ausführungsbeispiel aus Fig. 1 mit einem Deckel und einer zweiten Bodenkomponente.
- Fig. 7A bis 7C: einen beispielhaften ersten Konzentrationsgradienten an verschiedenen Stellen der Erstreckung einer Hydrogel-Matrix in z-Richtung.
- Fig. 8A bis 8C: einen beispielhaften zweiten Konzentrationsgradienten an verschiedenen Stellen der Erstreckung einer Hydrogel-Matrix in z-Richtung.

### Figurenbeschreibung

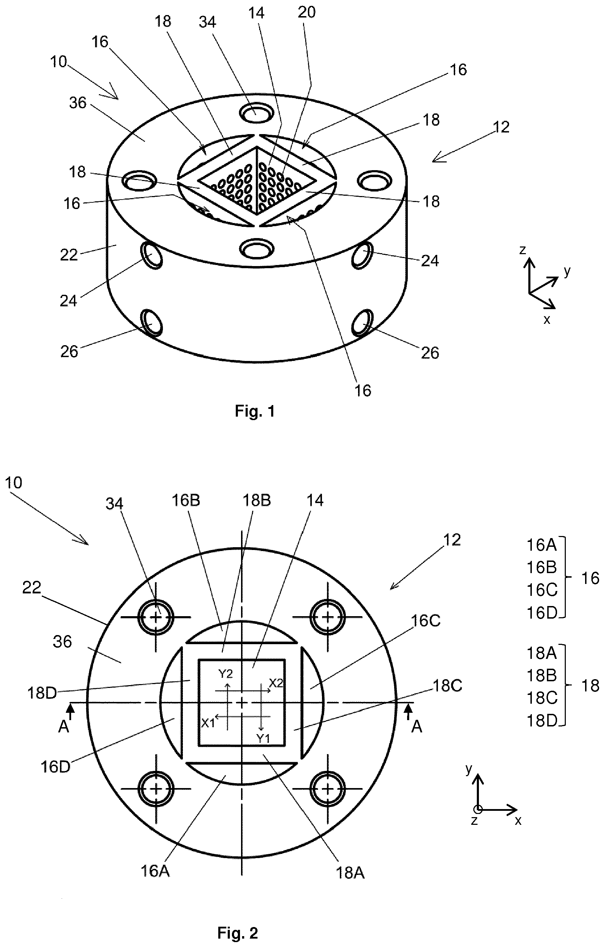

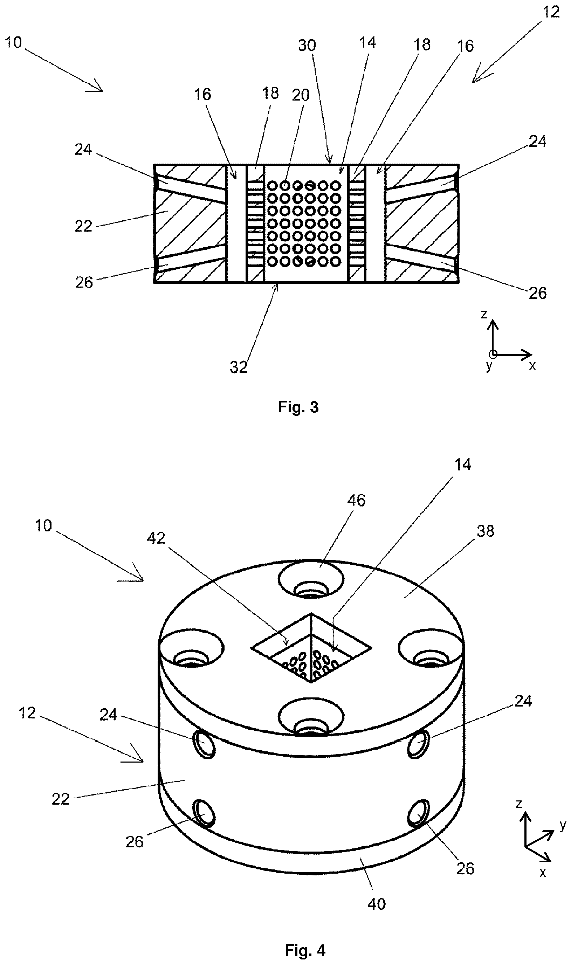

Die Figuren 1 bis 3 zeigen ein Ausführungsbeispiel der erfindungsgemäßen Fluidikvorrichtung 10 in verschiedenen Ansichten, wobei Fig. 1 eine perspektivische Ansicht, Fig. 2 eine Draufsicht und Fig. 3 eine Schnittansicht der Fluidikvorrichtung 10 entlang der Linie A-A in Fig. 2 zeigen.

Die Fluidikvorrichtung 10 hat einen Grundkörper 12, in dem eine Kammer 14 ausgebildet ist, die sich in einer x-Richtung, einer zu der x-Richtung orthogonalen y-Richtung und einer zu der x-Richtung und der y-Richtung orthogonalen z-Richtung erstreckt. In dem gezeigten Ausführungsbeispiel erstreckt sich die Kammer 14 mehrere Millimeter in jede der drei zueinander orthogonalen Richtungen.

Die Kammer 14 ist dafür vorgesehen eine Matrix (nicht gezeigt) aufzunehmen. Beispielsweise kann eine Hydrogel-Matrix, insbesondere eine Agarose-Matrix in die Kammer 14 eingebracht werden und diese vollständig ausfüllen. Die Matrix dient dazu, ein in die Matrix eingebrachtes zelluläres Ausgangsmaterial, welches mittels der Fluidikvorrichtung 10 zu einem dreidimensionalen zellulären Gebilde entwickelt werden soll, in einer vorgesehenen Position in der Kammer 14 anzuordnen und in dieser Position zu halten und gleichzeitig eine optimale Umgebung für die Entwicklung, das Größenwachstum, die Ausreifung und/oder die Erhaltung zu schaffen.

Der Grundkörper 12 der Fluidikvorrichtung 10 umfasst ferner vier Fluidreservoire 16, die voneinander getrennt und fluidisch vollständig voneinander isoliert sind. Jedes der vier Fluidreservoire 16 wird durch eine Trenneinrichtung 18 von der Kammer 14 getrennt, wobei die vier Trenneinrichtungen 18 miteinander verbunden sind und so die zwischen ihnen liegende Kammer 14 bilden. Mit anderen Worten begrenzt jede der Trenneinrichtungen 18 zu einer Seite hin ein zugehöriges Fluidreservoir 16, während die Trenneinrichtungen 18 zu ihrer jeweils anderen Seite hin gemeinsam die Kammer 14 begrenzen. Vorzugsweise bilden die vier Trenneinrichtungen 18 wie in dem gezeigten Beispiel eine dazwischenliegende Kammer 14, die eine quadratische Querschnittsfläche hat. Die Kammer 14 ist somit in dem gezeigten Beispiel ein Quader mit quadratischem Querschnitt. Die Trenneinrichtungen 18 sind in dem gezeigten Ausführungsbeispiel jeweils in Form einer flächigen Trennwand ausgebildet.

Jede der vier Trenneinrichtungen 18 ist teildurchlässig für in die Fluidreservoire 16 einbringbaren Fluidmedien. Die Trenneinrichtungen 18 stellen somit jeweils eine Schnittstelle zwischen einem zugehörigen der vier Fluidreservoire 16 und der Kammer 14 dar, über ein Fluidmedium aus jedem der Fluidreservoire 16 in die Kammer 14 hinein und aus der Kammer 14 heraus gelangen kann. Insbesondere können Fluidmedien aus den Fluidreservoiren 16 über die jeweils zugehörigen Trenneinrichtungen 18 in die in der Kammer 14 aufgenommene Matrix eindiffundieren und hierdurch Konzentrationsgradienten in der Matrix ausbilden.

In dem in den Figuren gezeigten Ausführungsbeispiel ist jede der Trenneinrichtungen 16 in Form einer perforierten Trennwand mit einer Vielzahl von Durchgangsöffnungen 20 ausgebildet. Der Übersicht halber ist in den Figuren maximal eine der Durchgangsöffnungen 20 mit einem Bezugszeichen versehen. In dem gezeigten Beispiel haben die Durchgangsöffnungen jeweils kreisförmige Querschnitte mit einem Durchmesser von etwa 0,5 mm. Die Anzahl, Geometrie und Größe und Anordnung der Durchgangsöffnungen kann in Abhängigkeit der verwendeten Matrix, in Abhängigkeit der verwendeten Fluidmedien, in Abhängigkeit der erwünschten Höhe der Konzentrationsdifferenzen und/oder in Abhängigkeit der erwünschten Homogenität der Gradienten gewählt werden, um einerseits zum Einstellen der vorgesehenen Diffusion eine angemessene Kontaktfläche zwischen der Matrix und den in den Fluidreservoiren 16 angestauten Fluidmedien sicherzustellen und andererseits ein Austreten der Matrix aus der Kammer 14 durch die Durchgangsöffnungen 20 zu verhindern.

Wie in den Figuren 1 bis 3 zu erkennen ist, erstrecken sich in dem gezeigten Ausführungsbeispiel zwei der flächigen Trenneinrichtungen 18, nämlich die Trenneinrichtungen 18A und 18B (siehe Fig. 2), jeweils ausschließlich in der z-Richtung und der x-Richtung. Diese zwei Trenneinrichtungen 18A, 18B sind einander gegenüberliegend angeordnet und durch die Kammer 14 in der y-Richtung voneinander beabstandet. Hierdurch kann bei einer Verwendung voneinander verschiedener Fluidmedien in den zugehörigen Fluidreservoiren 16A, 16B ein in der y-Richtung zwischen diesen zwei Trenneinrichtungen 18A, 18B verlaufender Konzentrationsgradient (Y1 oder Y2) oder zwei zueinander antiparallele in der y-Richtung zwischen diesen zwei Trenneinrichtungen verlaufende Konzentrationsgradienten (Y1 und Y2) gebildet werden. Lediglich aus Übersichtsgründen ist die zur Erläuterung dienende Unterscheidung der Fluidreservoire 16 durch die Bezugszeichen 16A bis 16D und die zur Erläuterung vorgesehene Unterscheidung der Trenneinrichtungen 18 durch die Bezugszeichen 18A bis 18D nur in Fig. 2 verwendet.

Ferner ist zu erkennen, dass sich in dem gezeigten Ausführungsbeispiel zwei weitere der Trenneinrichtungen 18, nämlich die Trenneinrichtungen 18C und 18D (siehe Fig. 2), jeweils ausschließlich in der z-Richtung und der y-Richtung erstrecken, wobei diese zwei weiteren Trenneinrichtungen 18C, 18D einander gegenüberliegend angeordnet und durch die Kammer 14 in der x-Richtung voneinander beabstandet sind. Hierdurch kann bei einer Verwendung voneinander verschiedener Fluidmedien in den zugehörigen Fluidreservoiren 16C, 16D ein in der x-Richtung zwischen diesen zwei Trenneinrichtungen 18C, 18D verlaufender Konzentrationsgradient (X1 oder X2) oder zwei zueinander antiparallele in der x-Richtung zwischen diesen zwei Trenneinrichtungen 18C, 18D verlaufende Konzentrationsgradienten (X1 und X2) gebildet werden.

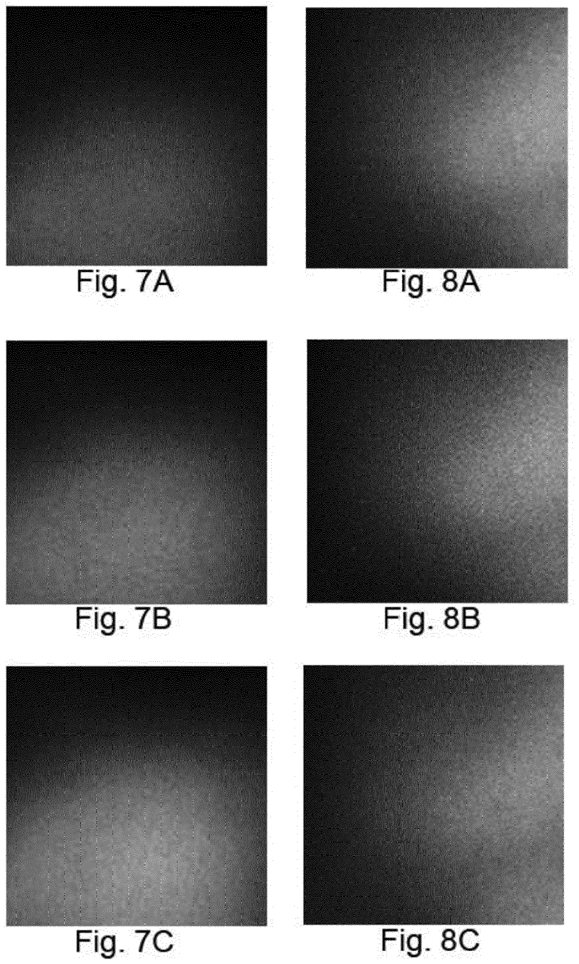

Somit können mittels der gezeigten Fluidikvorrichtung 10, bei Verwendung geeigneter Fluidmedien bis zu vier Konzentrationsgradienten in der Matrix ausgebildet werden, nämlich jeweils zwei Paare antiparalleler Konzentrationsgradienten (X1, X2 und Y1, Y2), wobei die Paare zueinander orthogonal sind. Es versteht sich, dass in bestimmten Anwendungen auch nur einer, zwei oder drei der gezeigten Konzentrationsgradienten X1, X2, Y1, Y2 ausgebildet werden kann. Aufgrund der Erstreckung der Kammer 14 bzw. der Trenneinrichtungen 18 in der z-Richtung, können alle der Konzentrationsgradienten X1, X2, Y1, Y2 über die Erstreckung der Matrix in der z-Richtung homogen ausgebildet werden. Dies ist beispielhaft in den Figuren 7A bis 7C und 8A bis 8C gezeigt, die nachstehend im Detail erläutert sind.

Der Grundkörper 12 hat in dem gezeigten Ausführungsbeispiel eine im Wesentlichen zylindrische Gestalt, wobei der Grundkörper 12 einen hülsenförmigen Mantel 22 aufweist. Jedes der vier Fluidreservoire 16 ist in dem Beispiel durch eine zugehörige Trenneinrichtung 18 und einen zugehörigen Abschnitt bzw. ein Segment der Innenumfangsfläche des hülsenförmigen Mantels 22 gebildet. Eine solche zylindrische Ausgestaltung des Grundkörpers 12 mit einem hülsenförmigen Mantel 22 kann insbesondere für die Fertigung der Fluidikvorrichtung 10 vorteilhaft sein. Beispielsweise können der hülsenförmige Mantel 22 und die die quaderförmige Kammer 14 (mit quadratischem Querschnitt) bildenden Trenneinrichtungen 18 in einem ersten Schritt separat voneinander hergestellt werden. Die Kammer 14 kann anschließend in die Innenausnehmung des hülsenförmigen Mantels 22 eingepresst werden, sodass die vier Längskanten der quaderförmigen Kammer 14 mit der Innenumfangsfläche des hülsenförmigen Mantels 22 in Berührung stehen, genauer gesagt eine Presspassung mit dieser bilden. Hierdurch werden die vier Fluidreservoire 16 fluidisch voneinander getrennt. Alternativ zur Herstellung und anschließender Zusammensetzung einzelner Teile kann das System in einem Schritt hergestellt werden, beispielsweise mittels 3D-Druck.

Jedes der Fluidikreservoire 16 ist mit einem in dem Grundkörper 12 ausgebildeten Fluideinlass 24 und einem in dem Grundkörper 12 ausgebildeten Fluidauslass 26 versehen (in Fig. 1 sind nur zwei Fluideinlässe 24 und zwei Fluidauslässe 26 zu erkennen). Die Fluideinlässe 24 erstrecken sich von außen durch den Mantel 22 des Grundkörpers 12 nach innen in die Fluidreservoire 16 hinein. Die Fluidauslässe 26 erstrecken sich von einer Innenseite der Fluidreservoire 16 durch den Mantel 22 des Grundkörpers 12 nach außen. Dies ist insbesondere aus Fig. 3 ersichtlich. Die Fluideinlässe 24 und Fluidauslässe 26 können dazu verwendet werden, die Fluidreservoire 18 kontinuierlich mit Fluidmedien zu durchströmen. Hierdurch kann ein kontinuierlicher Austausch von frischem und altem Fluidmedium sichergestellt werden, um eine optimale Nährstoffversorgung zu erreichen. Auf diese Weise können auch Abfallstoffe abgeführt werden, die in den Zellen entstehen und in den Reservoiren in niedriger Konzentration vorliegen. Bei Anschluss einer Überdruckpumpe an einer Seite, welche einen höheren Druck als an der gegenüberliegenden Seite generiert, werden solche Abfallstoffe vorzugsweise in eine Richtung abgeführt. Die Fluidikvorrichtung kann demnach als dynamisches System verwendet werden. Es versteht sich, dass die Fluideinlässe 24 und/oder Fluidauslässe 26 in weiteren Ausführungsbeispielen fluidisch mit Schläuchen verbunden sein können, um die verwendeten Fluidmedien besser zu- und/oder abzuführen. Die Schläuche können ferner mit einem Pumpensystem, beispielsweise einem Spritzenpumpensystem, verbunden sein, um die Flussrate in den einzelnen Fluidreservoiren 16 zu steuern. Vorzugsweise kann so in jedem der Fluidreservoire 16 eine Flussrate zwischen 20 und 50 µl/h eingestellt werden. Mittels des Pumpensystems kann hierzu an den Fluideinlässen 24 Überdruck und/oder an den Fluidauslässen 26 Unterdruck erzeugt werden.

Die Kammer 14 der dargestellten Fluidikvorrichtung 10 weist eine Eingangsöffnung 30 und eine Ausgangsöffnung 32 auf, zwischen denen sich die Kammer 14 in der z-Richtung erstreckt. Die Eingangsöffnung 30 und die Ausgangsöffnung 32 sind in z-Richtung betrachtet kongruent zueinander. Hierdurch kann die Kammer 14 mittels optischer Strahlen in der z-Richtung durchleuchtet werden, um eine Beobachtung und Analyse der entstehenden zellulären dreidimensionalen Gebilde mittels eines Mikroskops während der Entwicklung zu ermöglichen. Zudem ist die Fluidikvorrichtung 10 durch die Eingangsöffnung 30 und die Ausgangsöffnung 32 der Kammer 14 als ein offenes System ausgebildet, bei dem die Matrix in der Kammer 14 mit Umgebungsluft im Gasaustausch stehen kann.

Des Weiteren weist der Grundkörper 12 der Fluidikvorrichtung 10 in dem Ausführungsbeispiel an seinen Stirnseiten jeweils vier Bohrungen 34 auf, wobei in den dargestellten Figuren lediglich die Stirnseite 36 zu erkennen ist. Über die Bohrungen 34 an den Stirnseiten 36 kann der Grundkörper 12 der Fluidikvorrichtung 10 mit einem Deckel und/oder einer Bodenkomponente verbunden werden.

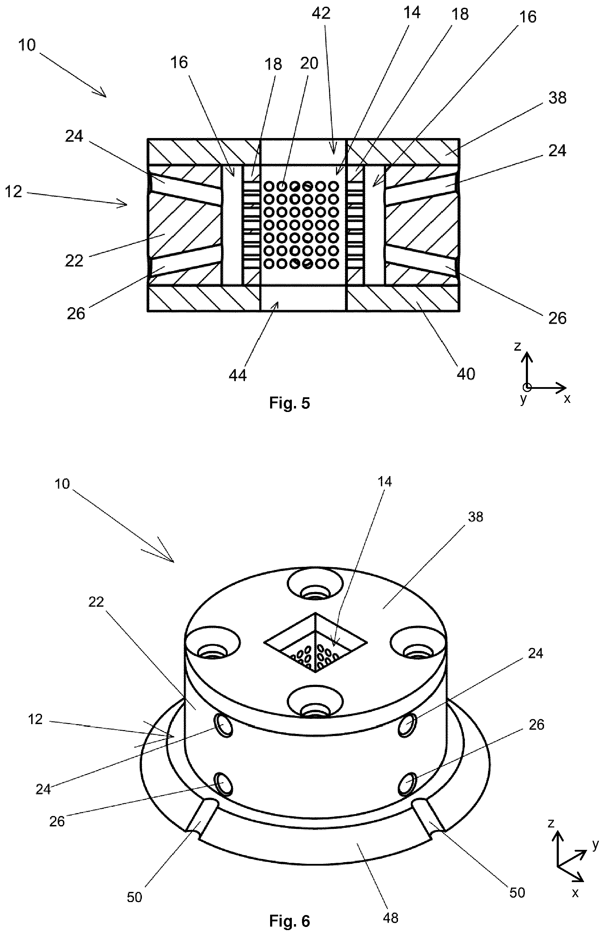

Fig. 4 zeigt ein erstes Ausführungsbeispiel eines mit dem Grundkörper 12 verbundenen Deckels 38 und einer mit dem Grundkörper 12 verbundenen Bodenkomponente bzw. Bodenplatte 40. Der Deckel 38 und die Bodenplatte 40 sind kongruent zu den Stirnseiten des Grundkörpers. Zudem sind der Deckel 38 und die Bodenplatte 40 in Fig. 4 einander entsprechend ausgebildet. Der Deckel 38 umfasst eine Deckelausnehmung 42, die in der z-Richtung mit der Eingangsöffnung 30 der Kammer 14 ausgerichtet ist. Entsprechend ist die Bodenplatte 40 mit einer Bodenausnehmung 44 (siehe Fig. 5) versehen, die in der z-Richtung mit der Ausgangsöffnung 32 der Kammer 14 ausgerichtet ist. Trotz Deckel 38 und Bodenplatte 40 kann die Fluidikvorrichtung 10 somit weiterhin ein offenes System darstellen, mit den vorstehend beschriebenen Vorteilen und Effekten.

Zur lösbaren Verbindung des Deckels 38 und der Bodenplatte 40 mit dem Grundkörper, weisen der Deckel 38 und die Bodenplatte 40 ebenfalls vier Bohrungen 46 auf. Der Deckel 38 kann mit dem Grundkörper 12 verbunden werden, indem die vier Bohrungen 46 des Deckels 38 mit den Bohrungen 34 des Grundkörpers 12 ausgerichtet werden. Anschließend können Schrauben oder Stifte in die Bohrungen eingebracht werden. Entsprechendes gilt für die Bodenplatte 40, obgleich die Bohrungen der Bodenkomponente in den Figuren nicht gezeigt sind.

Wie in der Schnittdarstellung in Fig. 5 zu erkennen ist, verschließen der Deckel 38 und die Bodenplatte 40 die vier Fluidreservoire 16 in der z-Richtung. Genauer gesagt dichtet der Deckel 38 die Fluidreservoire 16 in der z-Richtung zu einer Seite hin ab (in der gezeigten Darstellung nach oben hin), während die Bodenplatte 40 die Fluidreservoire 16 in der z-Richtung zu einer entgegengesetzten Seite hin abdichtet (in der gezeigten Darstellung nach unten hin). Somit können Fluidmedien nur über die Fluideinlässe 24, Fluidauslässe 26 und die teildurchlässigen Trenneinrichtungen 18 in die Fluidreservoire 16 bzw. aus den Fluidreservoiren 16 gelangen.

Fig. 6 zeigt ein zweites Ausführungsbeispiel eines mit dem Grundkörper 12 verbundenen Deckels 38 und einer mit dem Grundkörper 12 verbundenen Bodenkomponente bzw. Bodenplatte 48. Der Deckel 38 des zweiten Ausführungsbeispiels der Fig. 6 entspricht dem Deckel 38 des ersten Ausführungsbeispiels der Figuren 4 und 5. Jedoch unterscheiden sich die Bodenplatten dieser beiden Ausführungsbeispiele. Im Unterschied zu der Bodenplatte 40 des ersten Ausführungsbeispiels, ist die Bodenplatte 48 des zweiten Ausführungsbeispiels in Form eines Kegelstumpfes ausgebildet. Mit der Deckfläche des Kegelstumpfes grenzt die Bodenplatte 48 an die untere Stirnfläche des Grundkörpers 12 an. Hierdurch dient die Bodenplatte 48 auch als Fuß der Fluidikvorrichtung 10, der den Grundkörper 12 stabil gegenüber einem Untergrund abstützen kann. Die Deckfläche des Kegelstumpfes ist in dem gezeigten Beispiel geringfügig größer als die Fläche der angrenzenden Stirnseite des Grundkörpers 12. Jedoch können die Deckfläche des Kegelstumpfes und die Fläche der angrenzenden Stirnseite des Grundkörpers 12 in alternativen Ausführungsformen einander entsprechen.

Ein weiterer Unterschied zwischen der Bodenplatte 40 des ersten Ausführungsbeispiels der Figuren 4 und 5 und der Bodenplatte 48 des zweiten Ausführungsbeispiels der Fig. 6 besteht darin, dass die Bodenplatte 48 vier Rinnen 50 bzw. Nuten umfasst. Die Rinnen 50 sind in der Mantelfläche der kegelstumpfförmigen Bodenplatte 48 ausgebildet und sind in einem Zustand, in dem die Bodenplatte 48 mit dem Grundkörper 12 verbunden ist, jeweils benachbart zu einem Fluidauslässe 26 der Fluidreservoire 16 angeordnet. Mittels der Rinnen 50 können aus den Fluidauslässen 26 austretende Fluidmedien gezielt in einen oder mehrere benachbarte Auffangbehälter abgeführt werden.

Die Figuren 7A bis 7C und 8A bis 8C zeigen einen beispielhaften mittels der erfindungsgemäßen Fluidikvorrichtung 10 ausbildbaren homogenen Konzentrationsgradienten an verschiedenen Stellen der Erstreckung der Matrix in der z-Richtung. Genauer gesagt zeigen die Figuren 7A und 8A jeweils einen Konzentrationsgradienten in der Matrix in einem Abstand von 1 mm von einer unteren Stirnseite der Kammer 14. Die Figuren 7B und 8B zeigen jeweils einen Konzentrationsgradienten in der Matrix in einem Abstand von 2,5 mm von der unteren Stirnseite der Kammer 14. Die Figuren 7C und 8C zeigen jeweils einen Konzentrationsgradienten in der Matrix in einem Abstand von 4 mm von der unteren Stirnseite der Kammer 14. Die Figuren 7A bis 7C zeigen einen Konzentrationsgradienten, der in der Matrix gebildet wird, bei Verwendung eines Fluidmediums mit Alexa fluor 647-Dextran in dem Fluidreservoir 16A (siehe Fig. 2) und eines neutralen Fluidmediums in dem gegenüberliegenden Fluidreservoir 16B (siehe Fig. 2), die über die zugehörigen Trenneinrichtungen 18A und 18B in eine Agarose-Matrix eindiffundiert sind. Die Figuren 8A bis 8C zeigen einen Konzentrationsgradienten, der in der Hydrogel-Matrix gebildet wird, bei Verwendung eines Fluidmediums mit FITC-Dextran in dem Fluidreservoir 16C (siehe Fig. 2) und eines neutralen Fluidmediums in dem gegenüberliegenden Fluidreservoir 16D (siehe Fig. 2), die über die zugehörigen Trenneinrichtungen 18C und 18D in eine Agarose-Matrix eindiffundiert sind. Mit den verwendeten Farbstoffen wurde beispielhaft die Ausbildung zweier orthogonal zueinander liegender homogener Konzentrationsgradienten gezeigt. In analoger Art und Weise können biologischen Substanzen (z.B. Morphogene, Substanzen die morphogene Signalwege und/oder Differenzierungswege beeinflussen, Medikamente) zur Ausbildung eines Konzentrationsgradienten verwendet werden.

### Bezugszeichenliste

- 10: Fluidikvorrichtung
- 12: Grundkörper
- 14: Kammer
- 16, 16A, 16B, 16C, 16D: Fluidreservoir
- 18, 18A, 18B, 18C, 18D: Trenneinrichtung
- 20: Durchgangsöffnung
- 22: Mantel
- 24: Fluideinlass
- 26: Fluidauslass
- 30: Eingangsöffnung
- 32: Ausgangsöffnung
- 34: Bohrung
- 36: Stirnseite
- 38: Deckel
- 40: Bodenkomponente
- 42: Deckelausnehmung
- 44: Bodenausnehmung
- 46: Bohrung
- 48: Bodenkomponente
- 50: Rinne
- X1, X2: in der x-Richtung verlaufender Konzentrationsgradient
- Y1, Y2: in der y-Richtung verlaufender Konzentrationsgradient

## Patentansprüche

1. Fluidikvorrichtung (10) zum Entwickeln eines zellulären Ausgangsmaterials zu einem dreidimensionalen zellulären Gebilde, umfassend einen Grundkörper (12) mit
einer sich in einer x-Richtung, einer zu der x-Richtung orthogonalen y-Richtung und einer zu der x-Richtung und der y-Richtung orthogonalen z-Richtung erstreckenden Kammer (14), in der eine Matrix aufgenommen oder aufnehmbar ist, in die das zu entwickelnde Ausgangsmaterial einbringbar ist; und
wenigstens zwei Fluidreservoiren (16), wobei jedes der wenigstens zwei Fluidreservoire (16) aufweist:
einen Fluideinlass (24) zum Einbringen eines Fluidmediums in das zugehörige Fluidreservoir (16),
einen Fluidauslass (26) zum Auslassen des Fluidmediums aus dem zugehörigen Fluidreservoir (16), und
eine für das Fluidmedium teildurchlässige Trenneinrichtung (18), die das zugehörige Fluidreservoir (16) von der Kammer (14) trennt und eine gemeinsame flächige Schnittstelle des zugehörigen Fluidreservoirs (16) mit der Kammer (14) bildet, über die das Fluidmedium in die Matrix eindiffundieren kann;
wobei die Fluidikvorrichtung (10) dazu eingerichtet ist, bei Verwendung geeigneter Fluidmedien wenigstens einen Konzentrationsgradienten, wenigstens zwei zueinander orthogonale Konzentrationsgradienten und/oder wenigstens zwei zueinander antiparallele Konzentrationsgradienten (X1, X2, Y1, Y2) in der Matrix auszubilden, die jeweils über zumindest einen Abschnitt der Erstreckung der Matrix in der z-Richtung hinweg im Wesentlichen homogen oder gezielt inhomogen sind,
indem zwei der Trenneinrichtungen (18A, 18B) sich jeweils in der z-Richtung und der x-Richtung erstrecken und einander gegenüberliegend angeordnet und durch die Kammer (14) in der y-Richtung voneinander beabstandet sind, und/oder
indem zwei weitere der Trenneinrichtungen (18C, 18D) sich jeweils in der z-Richtung und der y-Richtung erstrecken und einander gegenüberliegend angeordnet und durch die Kammer (14) in der x-Richtung voneinander beabstandet sind.

2. Fluidikvorrichtung (10) nach Anspruch 1, wobei sich die Kammer (14) mehrere hundert µm, vorzugsweise wenigstens 1 mm, vorzugsweise mehrere Millimeter, vorzugsweise wenigstens 4 mm, vorzugsweise wenigstens 6 mm, vorzugsweise wenigstens 9 mm, weiter bevorzugt mehrere Zentimeter, noch weiter bevorzugt wenigstens 5 cm, noch weiter bevorzugt wenigstens 7 cm, noch weiter bevorzugt wenigstens 10 cm in der x-Richtung, der y-Richtung und/oder der z-Richtung erstreckt.

3. Fluidikvorrichtung (10) nach Anspruch 1 oder 2, wobei die Kammer (14) einen rechteckigen, vorzugsweise einen quadratischen, Querschnitt aufweist.

4. Fluidikvorrichtung (10) nach einem der vorangehenden Ansprüche, wobei die Trenneinrichtungen (18) miteinander verbunden sind und gemeinsam die zwischen ihnen liegende Kammer (14) bilden.

5. Fluidikvorrichtung (10) nach einem der vorangehenden Ansprüche, wobei jede der Trenneinrichtungen (18) in Form einer mit einer Vielzahl an Durchgangsöffnungen (20) versehenen Trennwand ausgebildet ist.

6. Fluidikvorrichtung (10) nach einem der vorangehenden Ansprüche, wobei die Kammer (14) eine Eingangsöffnung (30) und eine Ausgangsöffnung (32) umfasst, zwischen denen sich die Kammer (14) in der z-Richtung erstreckt, sodass die Kammer (14) mittels optischer Strahlen in der z-Richtung durchleuchtbar ist.

7. Fluidikvorrichtung (10) nach einem der vorangehenden Ansprüche, wobei die Matrix eine Hydrogel-Matrix, vorzugsweise eine Agarose-Matrix oder eine basalmembranartige Matrix ist.

8. Fluidiksystem zum Entwickeln eines zellulären Ausgangsmaterials zu einem dreidimensionalen zellulären Gebilde, umfassend eine Fluidikvorrichtung (10) nach einem der vorangehenden Ansprüche und ein Pumpensystem, wobei jeder Fluideinlass (24) über einen Einlassschlauch mit einer Pumpe zum Einbringen eines Fluidmediums in das zugehörige Fluidreservoir (16) verbunden ist, wobei die Pumpe vorzugsweise dazu eingerichtet ist, das Fluidmedium mit einer Flussrate zwischen 1 und 1000 µl/h, bevorzugt zwischen 1 und 100 µl/h, weiter bevorzugt zwischen 15 und 60 µl/h, noch weiter bevorzugt zwischen 20 und 50 µl/h durch den Fluideinlass (24) zu fördern,
wobei insbesondere jeder Fluidauslass (26) mit einem Auslassschlauch zum Auslassen des Fluidmediums aus dem zugehörigen Fluidreservoir (16) verbunden ist, und wobei der Auslassschlauch vorzugsweise mit einer Auslasspumpe zum Beaufschlagen des Fluidauslasses (26) mit einem vorbestimmten Unterdruck verbunden ist.

9. Verfahren zum Entwickeln von zellulärem Ausgangsmaterial zu einem oder mehreren dreidimensionalen zellulären Gebilden und/oder zu deren Reifung, Größenwachstum oder Aufrechterhaltung, wobei das Verfahren die Schritte umfasst:
Bereitstellen des zu entwickelnden Ausgangsmaterials in einer Matrix, die in einer Kammer einer Fluidikvorrichtung aufgenommen ist, wobei sich die Kammer und die Matrix in einer x-Richtung, einer zu der x-Richtung orthogonalen y-Richtung und einer zu der x-Richtung und der y-Richtung orthogonalen z-Richtung erstrecken, und wobei die Fluidikvorrichtung wenigstens zwei fluidisch voneinander getrennte Fluidreservoire aufweist,
wobei jedes der wenigstens zwei Fluidreservoire eine für das Fluidmedium teildurchlässige Trenneinrichtung umfasst, die das zugehörige Fluidreservoir von der Kammer trennt und eine gemeinsame flächige Schnittstelle des zugehörigen Fluidreservoirs mit der Kammer bildet, und wobei jeweils zwei der Trenneinrichtungen an einander entgegengesetzten Seiten der Matrix angeordnet sind;
Durchströmen der wenigstens zwei Fluidreservoire mit jeweils einem Fluidmedium;
Ausbilden wenigstens eines Konzentrationsgradienten, wenigstes zweier zueinander orthogonaler Konzentrationsgradienten und/oder wenigstens zweier antiparalleler Konzentrationsgradienten in der Matrix, die jeweils über zumindest einen Abschnitt der Erstreckung der Matrix in der z-Richtung hinweg im Wesentlichen homogen oder gezielt inhomogen sind, indem über die teildurchlässigen Trenneinrichtungen der wenigstens zwei durchströmten Fluidreservoire das jeweils zugehörige Fluidmedium über den Abschnitt der Erstreckung der Matrix in der z-Richtung hinweg in die Matrix eindiffundiert, wobei jeweils an einander entgegengesetzten Seiten der Matrix voneinander verschiedene Fluidmedien in die Matrix eindiffundieren.

## Claims

1. A fluidic device (10) for developing a cellular starting material into a three-dimensional cellular construction, comprising a base body (12) with
a chamber (14) extending in an x-direction, a y-direction being orthogonal to the x-direction and a z-direction being orthogonal to the x-direction and the y-direction, in which a matrix is accommodated or can be accommodated, into which the starting material to be developed can be introduced; and
at least two fluid reservoirs (16), wherein each of the at least two fluid reservoirs (16) comprises:
a fluid inlet (24) for introducing a fluid medium into the corresponding fluid reservoir (16),
a fluid outlet (26) for draining the fluid medium from the corresponding fluid reservoir (16), and
a separating means (18) being partially permeable for the fluid medium,
which separates the corresponding fluid reservoir (16) from the chamber (14) and forms a common planar interface of the corresponding fluid reservoir (16) with the chamber (14), via which the fluid medium can diffuse
into the matrix;
wherein the fluidic device (10) is configured to form, when suitable fluid media are used, at least one concentration gradient, at least two concentration gradients being orthogonal to each other and/or at least two concentration gradients being antiparallel to each other (X1, X2, Y1, Y2) in the matrix, which each are substantially homogeneous or purposefully inhomogeneous over at least a section of the extension of the matrix in the z-direction,
provided that two of the separating means (18A, 18B) each extend in the z-direction and the x-direction and are arranged opposite from each other and are spaced apart from one another in the y-direction by the chamber (14), and/or
provided that two more of the separating means (18C, 18D) each extend in the z-direction and the y-direction and are arranged opposite from each other and are spaced apart from one another in the x-direction by the chamber (14).

2. The fluidic device (10) according to claim 1, wherein the chamber (14) extends several hundred µm, preferably at least 1 mm, preferably several millimeters, preferably at least 4 mm, preferably at least 6 mm, preferably at least 9 mm, further preferably several centimeters, stiller further preferably at least 5 cm, still further preferably at least 7 cm, still further preferably at least 10 cm in the x-direction, the y-direction and/or the z-direction.

3. The fluidic device (10) according to claim 1 or 2, wherein the chamber (14) has a rectangular, preferably a square cross-section.

4. The fluidic device (10) according to one of the preceding claims, wherein the separating means (18) are connected with each other and together form the chamber (14) which is located between them.

5. The fluidic device (10) according to one of the preceding claims, wherein each of the separating means (18) is in the form of a separating wall which is provided with a plurality of through openings (20).

6. The fluidic device (10) according to one of the preceding claims, wherein the chamber (14) comprises an entry opening (30) and an exit opening (32), between which the chamber (14) extends in the z-direction, so that it is possible to ray the chamber (14) in the z-direction by means of optical radiation.

7. The fluidic device (10) according to one of the preceding claims, wherein the matrix is a hydrogel matrix, preferably an agarose matrix or a basal membrane-like matrix.

8. A fluidic system for developing a cellular starting material into a three-dimensional cellular construction, comprising a fluidic device (10) according to one of the preceding claims and a pump system, wherein each fluid inlet (24) is connected via an inlet tube with a pump for introducing a fluid medium into the corresponding fluid reservoir (16), wherein the pump is preferably configured to deliver the fluid medium through the fluid inlet (24) with a flow rate of between 1 and 1000 µl/h, preferably between 1 and 100 µl/h, further preferably between 15 and 60 µl/h, still further preferably between 20 and 50 µl/h,
wherein in particularly each fluid outlet (26) is connected with an outlet tube for draining the fluid medium from the corresponding fluid reservoir (16), and wherein the outlet tube is preferably connected with an outlet pump for applying a predetermined negative pressure to the fluid outlet (26).

9. A method for developing cellular starting material into one or more three-dimensional cellular constructions and/or for their maturation, size growth or maintenance, wherein the method comprises the steps:
providing of the starting material to be developed in a matrix, which is accommodated in a chamber of a fluidic device, wherein the chamber and the matrix extend in an x-direction, a y-direction being orthogonal to the x-direction and a z-direction being orthogonal to the x-direction and the y-direction, and wherein the fluidic device comprises at least two fluid reservoirs which are fluidly separated from each other, wherein each of the at least two fluid reservoirs comprises a separating means being partially permeable for the fluid medium, which separates the corresponding fluid reservoir from the chamber and forms a common planar interface of the corresponding fluid reservoir with the chamber, and wherein two of the separating means each are arranged on sides of the matrix which are opposite to each other;
flowing of one fluid medium each through the at least two fluid reservoirs;
forming of at least one concentration gradient, at least two concentration gradients being orthogonal to each other and/or at least two antiparallel concentration gradients in the matrix, which each are substantially homogeneous or purposefully inhomogeneous over at least a section of the extension of the matrix in the z-direction, provided that via the partially permeable separating means of the at least two flown-through fluid reservoirs the respective corresponding fluid medium diffuses over the section of the extension of the matrix in the z-direction into the matrix, wherein respectively on sides of the matrix which are opposite to each other fluid media which are different from each other diffuse into the matrix.

## Revendications

1. Dispositif fluidique (10) destiné à développer un matériau de départ cellulaire en une structure cellulaire tridimensionnelle, comprenant un corps de base (12) possédant
une chambre (14) qui s'étend dans une direction x, une direction y orthogonale à la direction x et une direction z orthogonale à la direction x et à la direction y et dans laquelle est reçue ou peut être reçue une matrice dans laquelle peut être introduit le matériau de départ à développer ; et
au moins deux réservoirs de fluide (16), chacun des au moins deux réservoirs de fluide (16) comportant :
une entrée de fluide (24) destinée à introduire un milieu fluide dans le réservoir de fluide associé (16),
une sortie de fluide (26) destinée à évacuer le milieu fluide du réservoir de fluide associé (16), et
un moyen de séparation (18) qui est partiellement perméable au milieu fluide et qui sépare le réservoir de fluide (16) associé de la chambre (14) et qui forme entre le réservoir de fluide (16) associé et la chambre (14) une interface commune plane, permettant au milieu fluide
de diffuser dans la matrice ;
dans lequel le dispositif fluidique (10) est adapté pour former, lors de l'utilisation de milieux fluides appropriés, au moins un gradient de concentration, au moins deux gradients de concentration orthogonaux l'un à l'autre et/ou au moins deux gradients de concentration antiparallèles (X1, X2, Y1, Y2) dans la matrice, qui sont sensiblement homogènes ou de manière ciblée inhomogènes dans la direction z à chaque fois sur au moins une portion de l'extension de la matrice,
du fait que deux des moyens de séparation (18A, 18B) s'étendent chacun dans la direction z et la direction x et sont disposés l'un en face de l'autre et sont espacés l'un de l'autre par la chambre (14) dans la direction y, et/ou
du fait que deux autres moyens de séparation (18C, 18D) s'étendent chacun dans la direction z et la direction y et sont disposés l'un en face de l'autre et sont espacés l'un de l'autre par la chambre (14) dans la direction x.

2. Dispositif fluidique (10) selon la revendication 1, dans lequel la chambre (14) s'étend sur plusieurs centaines de µm, de préférence sur au moins 1 mm, de préférence sur plusieurs millimètres, de préférence sur au moins 4 mm, de préférence sur au moins 6 mm, de préférence sur au moins 9 mm, plus préférablement sur plusieurs centimètres, encore plus préférablement sur au moins 5 cm, encore plus préférablement sur au moins 7 cm, encore plus préférablement sur au moins 10 cm dans la direction x, la direction y et/ou la direction z.

3. Dispositif fluidique (10) selon la revendication 1 ou 2, dans lequel la chambre (14) a une section transversale rectangulaire, de préférence carrée.

4. Dispositif fluidique (10) selon l'une des revendications précédentes, dans lequel les moyens de séparation (18) sont reliés entre eux et formant conjointement la chambre (14) située entre eux.

5. Dispositif fluidique (10) selon l'une des revendications précédentes, dans lequel chacun des moyens de séparation (18) est sous la forme d'une paroi de séparation munie d'une pluralité d'ouvertures traversantes (20).

6. Dispositif fluidique (10) selon l'une des revendications précédentes, dans lequel la chambre (14) comprend une ouverture d'entrée (30) et une ouverture de sortie (32) entre lesquelles la chambre (14) s'étend dans la direction z de sorte que la chambre (14) puisse être éclairée dans la direction z par la traversée de faisceaux optiques.

7. Dispositif fluidique (10) selon l'une des revendications précédentes, dans lequel la matrice est une matrice d'hydrogel, de préférence une matrice d'agarose ou une matrice de type membrane basale.

8. Système fluidique destiné à développer un matériau de départ cellulaire en une structure cellulaire tridimensionnelle, comprenant un dispositif fluidique (10) selon l'une des revendications précédentes et un système de pompe, dans lequel chaque entrée de fluide (24) est reliée par un tuyau d'entrée à une pompe d'introduction d'un milieu fluide dans le réservoir de fluide (16) associé, dans lequel la pompe est de préférence conçue pour transporter le milieu fluide à un débit compris entre 1 et 1000 µl/h, de préférence entre 1 et 100 µl/h, plus préférablement entre 15 et 60 µl/h, encore plus préférablement entre 20 et 50 µl/h par l'entrée de fluide (24),
dans lequel en particulier chaque sortie de fluide (26) étant reliée à un tuyau de sortie destiné à évacuer le milieu fluide du réservoir de fluide associé (16), et dans lequel le tuyau de sortie est de préférence relié à une pompe de sortie destinée à soumettre la sortie de fluide (26) à une pression négative prédéterminée.

9. Procédé de développement d'un matériau de départ cellulaire pour former une ou plusieurs structures cellulaires tridimensionnelles et/ou pour les amener à maturation, les amener à croître en taille ou à les maintenir, le procédé comprenant les étapes suivante :
fournir le matériau de départ à développer dans une matrice qui est reçue dans une chambre d'un dispositif fluidique, dans lequel la chambre et la matrice s'étendent dans une direction x, une direction y orthogonale à la direction x et une direction z orthogonale à la direction x et à la direction y, et dans lequel le dispositif fluidique comporte au moins deux réservoirs de fluide séparés fluidiquement l'un de l'autre, chacun des au moins deux réservoirs de fluide comprend un moyen de séparation qui est partiellement perméable au milieu fluide et qui sépare le réservoir de fluide associé de la chambre et forme une interface commune plane, entre le réservoir de fluide associé et la chambre, et dans lequel deux des moyens de séparation sont disposés respectivement sur des côtés opposés de la matrice ;
amener un milieu fluide à s'écouler à travers chacun des au moins deux réservoirs de fluide ;
former au moins un gradient de concentration, au moins deux gradients de concentration orthogonaux l'un à l'autre et/ou au moins deux gradients de concentration antiparallèles dans la matrice, qui sont respectivement sensiblement homogènes ou de manière ciblée inhomogènes sur au moins une portion de l'étendue de la matrice dans la direction z du fait que le milieu fluide à chaque fois associé diffuse dans la matrice par le biais des moyens de séparation partiellement perméables des au moins deux réservoirs de fluide traversés sur la portion de l'extension de la matrice dans la direction z, différents milieux fluides diffusant dans la matrice sur respectivement des côtés opposés de la matrice.
